(19) Europäisches Patentamt
European Patent Office
Office européen des brevets

(11) Veröffentlichungsnummer: **0 378 102 A2**

(12) # EUROPÄISCHE PATENTANMELDUNG

(21) Anmeldenummer: **90100138.8**

(51) Int. Cl.5: **A61L 27/00**

(22) Anmeldetag: **04.01.90**

(30) Priorität: **12.01.89 DE 3900708**

(43) Veröffentlichungstag der Anmeldung:
**18.07.90 Patentblatt 90/29**

(84) Benannte Vertragsstaaten:
**BE DE FR GB NL SE**

(71) Anmelder: **BASF Aktiengesellschaft**
**Carl-Bosch-Strasse 38**
**D-6700 Ludwigshafen(DE)**

(72) Erfinder: **Pohlemann, Heinz, Dr.**
**Hardenburgstrasse 25**
**D-6703 Limburgerhof(DE)**
Erfinder: **Hisgen, Bernd, Dr.**
**Goethestrasse 6**
**D-6703 Limburgerhof(DE)**

(54) **Implantatmaterialien.**

(57) Implantatmaterial zum Ersatz von hartem Gewebe, enthaltend als wesentliche Komponenten
A) 30 bis 95 Gew.% eines thermotrop mesomorphen Polymeren
B) 5 bis 70 Gew.% eines Apatits
C) 0 bis 60 Gew.% eines von B) verschiedenen faser- oder teilchenförmigen Füllstoffs.

EP 0 378 102 A2

Xerox Copy Centre

## Implantatmaterialien

Die Erfindung betrifft ein Implantatmaterial zum Ersatz von hartem Gewebe im Körper, enthaltend als wesentliche Komponenten

A) 30 bis 95 Gew.% eines thermotrop mesomorphen Polymeren,

B) 5 bis 70 Gew.% eines Apatits und

C) 0 bis 60 Gew.% von B) verschiedene faser- oder teilchenförmige Füllstoffe oder deren Mischungen.

Als Folge von chirurgischen oder orthopädischen Behandlungen, die einen Verlust von Knochen- oder Gelenkteilen zur Folge hatten, sind häufig prothetische Operationen erforderlich, um einen Ersatz der entfernten Teile vorzunehmen.

Hauptbestandteile von Knochen im menschlichen und tierischen Körper sind Kollagen (organisches Material) und Apatit (anorganisches Material).

Als Implantatmaterialien zum Ersatz von hartem Gewebe sind bislang natürliche Materialien, metallische oder keramische Werkstoffe und - in neuerer Zeit - auch Systeme auf der Basis von verstärkten thermoplastisch verarbeitbaren Polymeren eingesetzt worden.

Natürliche Materialien wie Elfenbein oder tierische Knochen sind zwar häufig mit dem Gewebe, in das sie eingepflanzt werden sollen, gut verträglich, doch sind deren mechanische Eigenschaften nur schwer im voraus abzuschätzen und nur schlecht reproduzierbar.

Metallische, keramische oder polymere Materialien sind häufig mit biologischen Materialien nur in geringem Maße verträglich, was insbesondere beim Einbau der Prothese zu Entzündungen und Infektionen führen kann.

Es sind auch Implantatmaterialien bekannt, die aus einer Kombination aus metallischen bzw. keramischen Werkstoffen und Polymeren aufgebaut sind, wobei letztere insbesondere die Versiegelung der Materialoberfläche bewirken soll, was zu einem niedrigen Reibungskoeffizienten führt. Die Befestigung am Knochen wird häufig durch in situ Polymerisation bewerkstelligt, was aufgrund der dabei entstehenden lokalen Erhitzung zu schwerwiegenden Entzündungen und Gewebeschädigungen führen kann.

Da Knochen - wie bereits erwähnt - im wesentlichen aus Kollagen und Apatit bestehen, hat es nicht an Versuchen gefehlt, Implantatmaterialien zu entwickeln, die diese Materialien enthalten.

Gesinterte Apatite haben aufgrund ihrer Verträglichkeit mit biologischem Gewebe Interesse als Knochen- und Zahnersatz gefunden, doch stehen ihrer breiten Anwendung die hohe Sprödigkeit und die ungenügende mechanische Festigkeit im Weg (vgl. H. Aoki et al, Ceramics 10, 57-66 (1975)).

In der DE-A 28 21 354 werden Verbundimplantatmaterialien aus einem keramischen Körper als kontinuierlicher Phase und mit Kunstharz gefüllten Löchern beschrieben, die zwar gegenüber gesinterten Apatiten hinsichtlich mechanischer Eigenschaften gewisse Vorteile bieten, jedoch noch nicht in vollem Umfang zufriedenstellen. Außerdem ist das Herstellungsverfahren der Produkte, die ja gesinterten Apatait enthalten, relativ aufwendig.

In der WO 82/01310 ist ein Kompositmaterial beschrieben, welches aus einem Homo- oder Copolyolefin mit einem Gewichtsmittel des Molekulargewichts von mehr als 20 000 und bis zu 80 Gew.% eines teilchenförmigen anorganischen Füllstoffs besteht. Als bevorzugtes Beispiel für den anorganischen Füllstoff wird natürlicher oder synthetischer Hydroxyapatit erwähnt.

Derartige Materialien weisen zwar eine gute Reproduzierbarkeit der mechanischen Eigenschaften auf, doch sind insbesondere Festigkeit und Steifigkeit noch verbesserungswürdig.

Auch die DE-A 35 42 535 betrifft Implantatmaterialien zum Ersatz von hartem Gewebe im lebenden Körper. Diese bestehen aus 10 bis 90 Gew.% Glasfasern, die überwiegend aus Calciumphosphat bestehen, und 10 bis 90 Gew.% organischen Polymeren, wobei insbesondere Polyethylen, Polypropylen, Polymethylmethacrylat and Poly(trifluorethylmethacrylat) bevorzugt werden; die beiden letztgenannten Polymeren sollen insbesondere zu guten Festigkeiten führen, die jedoch ebenfalls noch nicht voll zufriedenstellend sind.

Aufgabe der vorliegenden Erfindung war es daher, Implantatmaterialien zum Ersatz von hartem Gewebe im Körper zur Verfügung zu stellen, welches zum einen mit biologischem Gewebe gut verträglich ist und dessen mechanische Eigenschaften gut reproduzierbar sind. Inbesondere sollten Steifigkeit und Festigkeit gegenüber bekannten Materialien auf Polymerbasis verbessert werden.

Diese Aufgabe wird erfindungsgemäß durch die eingangs definierten Implantatmaterialien gelöst.

Als Komponente A) enthalten die erfindungsgemäßen Implantatmaterialien 30 bis 95, vorzugsweise 35 bis 90 und insbesondere 40 bis 75 Gew.% eines thermotrop mesomorphen Polymeren. Diese wurden in den letzten Jahren in einer Vielzahl von Veröffentlichungen und Patentanmeldungen beschrieben.

Thermotrop mesomorphe Polymere weisen eine anisotrope Schmelzphase auf, die leicht nach dem in der DE-A 25 20 819 beschriebenen Verfahren mit dem Polarisationsmikroskop nachgewiesen werden kann. Zwischen gekreuzten Polarisatoren weisen die Polymerschmelzen, die in einer Schichtdicke von 10 µm zwischen Glasplatten aufgetragen sind, Texturen auf, die einer mesomorphen Phase zugeordnet werden können.

Zur Erzielung einer anisotropen (flüssig-kristallinen) Schmelzphase ist in der Regel ein bestimmtes Maß an Linearität in der Hauptkette erforderlich, welche durch entsprechende Wahl der Mengenverhältnisse der Monomeren erzielt werden kann. Die Anisotropie der Schmelzphase und die damit einhergehende Orientierung der Polymermoleküle führen zu sehr hohen Festigkeiten und Steifigkeiten in aus derartigen Polymeren hergestellten Formkörpern.

Es sei betont, daß sich prinzipiell zur Herstellung der erfindungsgemäßen Implantatmaterialien als Komponente A) alle thermotrop mesomorphen Polymeren, unabhängig von der Zusammensetzung, eignen.

Allgemein läßt sich sagen, daß thermotrop mesomorphe Polymere im allgemeinen Einheiten enthalten, die sich ableiten von

$a_1$) aromatischen oder aliphatischen Dicarbonsäuren,

$a_2$) aromatischen oder aliphatischen Diolen, Diaminen oder entsprechenden Monomeren mit einer Amino- und einer Hydroxylgruppe,

$a_3$) aromatischen Hydroxy- und Aminocarbonsäuren und

$a_4$) aromatischen Thiocarbonsäuren bzw. Dithiolen oder Thiophenolen.

Aus diesen Monomeren lassen sich durch entsprechende Kombination z.B. Polyester, Polyesteramide, Polyesterimide, Polyestercarbonate, Polyetherester, Polyetheresteramide, Polyesteramidimide, Polyestercarbamide und Polyetheresterimide herstellen.

Die Zusammensetzung dieser Produkte kann in weiten Bereichen schwanken und es sind eine Vielzahl von Monomeren einsetzbar. Wesentlich ist, daß die Polymere thermotrop mesomorphe Eigenschaften aufweisen, was leicht nach dem bereits erwähnten, in der DE-A 25 20 819 beschriebenen Verfahren zu überprüfen ist.

Nicht alle Kombinationen von Monomeren $a_1$) bis $a_4$) in beliebigen molaren Verhältnissen führen zu thermotrop mesomorphen Polymeren, eine allgemeine Angabe von geeigneten Mengenverhältnissen ist kaum möglich.

In der einschlägigen Literatur und in einer Vielzahl von Patentanmeldungen sind jedoch geeignete thermotrop mesomorphe Systeme beschrieben, die nun nachstehend zunächst über eine beispielhafte Aufzählung geeigneter Monomerer und entsprechender Polymerer näher erläutert werden sollen.

Monomere $a_1$:

Terephthalsäure, Isophthalsäure, 2,6-Naphthalindicarbonsäure, 2,7-Naphthalindicarbonsäure, 4,4′-Dicarboxydiphenyl, 4,4″-Dicarboxyterphenyl, Dicarboxydiphenylderivate der allgemeinen Formeln I und II

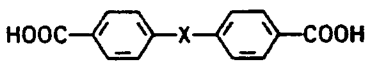

(I)

| | |
|---|---|
| X = O | 4,4'-Dicarboxydiphenylether |
| X = $CH_2CH_2$ | 1,2-Di-(4-carboxyphenyl)ethan |
| X = $OCH_2CH_2O$ | 1,2-Di-(4-carboxyphenoxy)-ethan |
| X = $O(CH_2)_4O$ | 1,4-Di-(4-carboxyphenoxy)-butan |

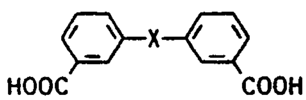

(II)

| | |
|---|---|
| X = O | 3,3'-Dicarboxydiphenylether |
| X = $CH_2CH_2$ | 1,2-Di-(3-carboxyphenyl)ethan |
| X = $OCH_2CH_2O$ | 1,2-Di-(3-carboxyphenoxy)-ethan |
| X = $O(CH_2)_4O$ | 1,4-Di-(3-carboxyphenoxy)-butan |

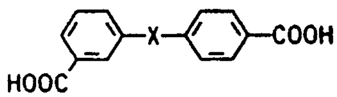

| | |
|---|---|
| X = O | 3,4'-Dicarboxydiphenylether |
| X = $CH_2CH_2$ | 1,2-(3,4'-Dicarboxy)-diphenyl-ethan |
| X = $OCH_2CH_2O$ | 1,2-(3,4-Dicarboxy)-diphenoxy-ethan |
| X = $O(CH_2)_4O$ | 1,4-(3,4'-Dicarboxy)-diphen-oxybutan |

Weiter sind zu nennen Dicarbonsäuren der allgemeinen Formel III und IV

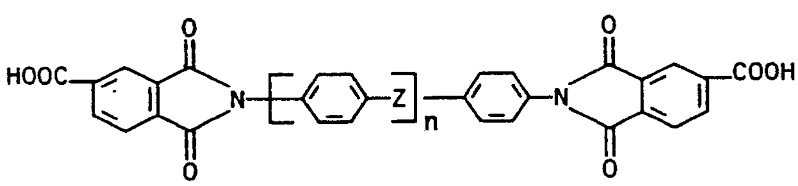

(III)

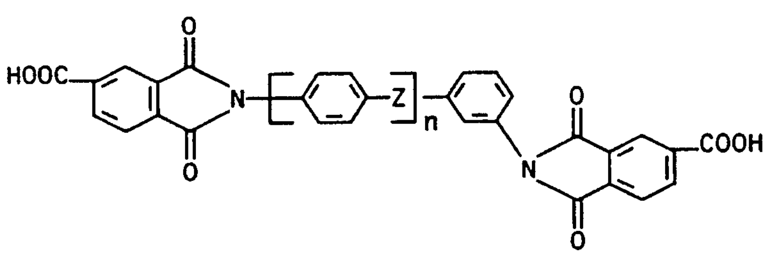

(IV)

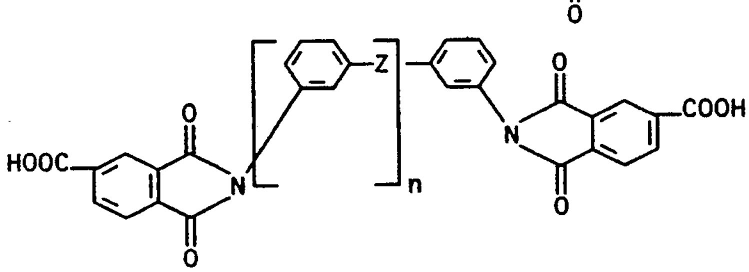

(V)

wobei Z jeweils -O-, -S-, $-SO_2-$, $-CH_2-$, $-C(CH_3)_2$ oder eine chemische Bindung und n 0 oder 1 ist.

Beispiele hierfür sind

Z = O 4,4'-, 3,4'- bzw. 3,3'-Di-(4-carboxy-N-phthalimido)diphenylether

Z = $CH_2$ 4,4'-, 3,4'- bzw. 3,3'-Di-(4-carboxy-N-phthalimido)diphenylmethan

Z = $SO_2$ 4,4′-, 3,4′- bzw. 3,3′-Di-(4-carboxy-N-phthalimido)diphenylsulfon
Z = CO 4,4′-, 3,4′- bzw. 3,3′-Di-(4-carboxy-N-phthalimido)diphenylketon
Z = S 4,4′-, 3,4′- bzw. 3,3′-Di-(4-carboxy-N-phthalimido)diphenylsulfid und z.B.
Z = $C(CH_3)_2$ 2,2-Di-[4,4′-di(4-carboxy-N-phthalimido)diphenyl]propan.

Ebenfalls geeignet sind p,p′-, m,m′- und p,m′-Dicarboxydiphenylcarbonate der allgemeinen Formeln VI bis VIII

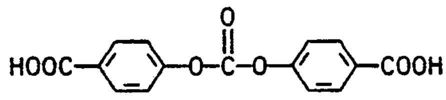 (VI)

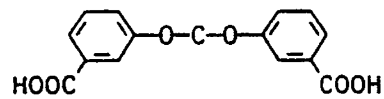 (VII)

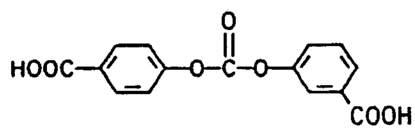 (VIII).

Die vorstehend genannten Carbonsäuren können auch Substituenten wie $C_1$-$C_4$-Alkyl-, $C_1$-$C_4$-Alkoxygruppen oder Halogenatome aufweisen. Schließlich seien auch noch einige aliphatische Dicarbonsäuren wie cis- und trans-1,4-Cyclohexandicarbonsäure und 1,3-Cyclohexandicarbonsäure sowie deren entsprechend substituierte Derivate erwähnt.

Monomere $a_2$:

Hydrochinon, Methylhydrochinon, Phenylhydrochinon, tert.-Butylhydrochinon, Chlorhydrochinon, 4,4′-Dihydroxydiphenyl, 1,4-Di-(4-hydroxyphenyl)benzol, 1,2-Di-(4-hydroxyphenoxy)ethan, 4,4′-Dihydroxydiphenylether, 4,4′-Dihydroxydiphenylsulfon, 3,3′-Dihydroxydiphenyl, 3,3′-Dihydroxydiphenylether, 3,4′-Dihydroxydiphenyl, 3,4′-Dihydroxydiphenylether, 2,2-Di-(4-hydroxyphenyl)propan, 1,6-, 2,6- und 2,7-Dihydroxynaphthalin, 3,3′,5,5′-Tetramethyl-4,4′-dihydroxydiphenyl, 4,4′-(Di(p-hydroxyphenoxy)-diphenylsulfon, Harnstoff, 1,4-Diaminobenzol, 1,3-Diaminobenzol, 3-Aminophenol, 4-Aminophenol, trans- und cis-1,4-Cyclohexandiol, trans-1,3-Cyclohexandiol and cis-1,2-Cyclohexandiol. Es versteht sich, daß auch hier wieder allgemein Substituenten wie bei den Monomeren $a_1$) vorhanden sein können.

Monomere $a_3$:

4-Hydroxybenzoesäure, 3-Hydroxybenzoesäure, 6-Hydroxynaphthalin-2-carbonsäure, 6-Hydroxynaphthalin-1-carbonsäure, 3-Aminobenzoesäure, 4-Aminobenzoesäure und deren $C_1$-$C_4$-Alkyl-, $C_1$-$C_4$-Alkoxy- oder Halogenderivate wie 3-Methyl-4-hydroxybenzoesäure, 3,5-Dimethyl-4-hydroxybenzoesäure, 2,6-Dimethyl-4-hydroxybenzoesäure, 3-Methoxy-4-hydroxybenzoesäure und 2,5-Dichlor-4-hydroxybenzoesäure um nur einige Beispiele zu nennen.

Monomere $a_4$:

4-Mercaptobenzoesäure, 3-Mercaptobenzoesäure, 6-Mercaptonaphthalin-2-carbonsäure, 2,7-Dimercaptonaphthalin, 2,6-Dimercaptonaphthalin, 1,4-Dimercaptobenzol und 1,3-Dimercaptobenzol sowie deren $C_1$-$C_4$-Alkyl-, $C_1$-$C_4$-Alkoxy- und Halogenderivate.

Weitere geeignete Monomere aus den Gruppen $a_1$) bis $a_4$) sind z.B. in der EP-A 206 600 aufgeführt.

Nachstehend werden eine Reihe bevorzugter thermotrop mesomorpher Polymerer vorgestellt.

1. Thermotrop mesomorphe Polyester auf

1a) 10 bis 25 Mol.% wiederkehrenden Einheiten der Formel

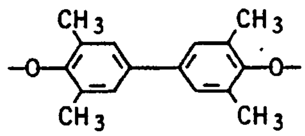

Als Ausgangsstoff wird vorteilhaft 3,3′,5,5′-Tetramethyl-4,4′-dihydroxydiphenyl verwendet.
1b) 5 bis 15 Mol.% wiederkehrenden Einheiten der Formel

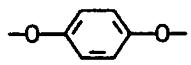

Als bevorzugte Ausgangsverbindung wird Hydrochinon verwendet.
1c) 5 bis 15 Mol.% wiederkehrenden Einheiten der Formel

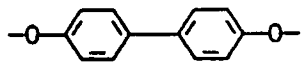

Als Ausgangsverbindung wird z.B. 4,4′-Dihydroxydiphenyl verwendet.
1d) mindestens 10 Mol.% wiederkehrenden Einheiten der Formel

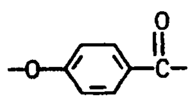

Als bevorzugte Ausgangsverbindung wird 4-Hydroxybenzoesäure verwendet.
1e) einer der Summe aus 1a), 1b), und 1c) entsprechenden molaren Menge an wiederkehrenden Einheiten der Formel

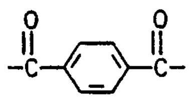

Als geeignete Ausgangsverbindung wird z.B. Terephthalsäure verwendet.

In bevorzugten vollaromatischen Polyestern dieses Typs ist ein Teil der Einheiten b) und/oder c), ersetzt durch
1f) wiederkehrende Einheiten der Formel

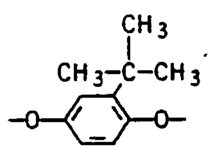

Ein geeigneter Ausgangsstoff hierfür ist z.B. tert.-Butylhydrochinon
und/oder
1g) wiederkehrenden Einheiten der Formel

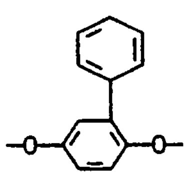

Als bevorzugte Ausgangsverbindung wird Phenylhydrochinon verwendet.

Vorteilhaft enthalten solche vollaromatischen Polyester die wiederkehrenden Einheiten 1f) und/oder 1g) in einer Menge von 2 bis 20 Mol.%. Ferner hat es sich als vorteilhaft erwiesen, wenn die Summe der molaren Anteile der Einheiten 1a) und 1f) und/oder 1g) von 20 bis 40 Mol.% beträgt.

In anderen bevorzugten vollaromatischen Polyestern ist ein Teil der Einheiten 1b) und/oder 1c) ersetzt durch

1h) wiederkehrende Einheiten der Formel

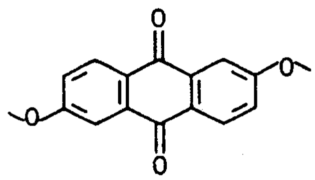

1i) wiederkehrenden Einheiten der Formel

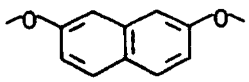

1j) wiederkehrenden Einheiten der Formel

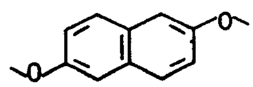

und/oder

1k) wiederkehrenden Einheiten der Formel

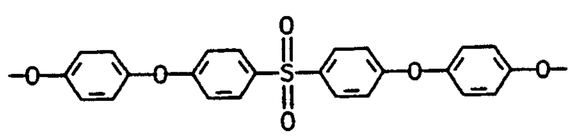

Als Ausgangsstoff für die Komponente 1h) wird 2,6-Dihydroxyanthrachinon, für die Komponente 1i) 2,7-Dihydroxynaphthalin, für die Komponente 1j) 2,6-Dihydroxynaphthalin und für die Komponente 1k) 4,4′-Di-(p-hydroxyphenoxy)diphenylsulfon verwendet.

Es hat sich bewährt, wenn an den erfindungsgemäßen Polyestern die Summe der molaren Anteile an den Einheiten 1a), 1h), 1i), 1j) and 1k) 20 bis 40 Mol.% beträgt.

Auf die Summe der Dihydroxyverbindungen wird vorteilhaft jeweils die äquivalente Menge Terephthalsäure verwendet.

Derartige Polyester sind in der EP-A-226-839 beschrieben.

2. Thermotrop mesomorphe Polyester aus

2a) 5 bis 35 Mol.% wiederkehrenden Einheiten der Formel

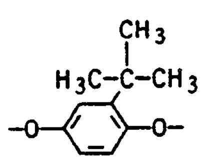

Als Ausgangsverbindung verwendet man t-Butylhydrochinon.

2b) 3 bis 15 Mol.% wiederkehrenden Einheiten der Formel

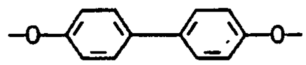

Als Ausgangsverbindung verwendet man 4,4′-Dihydroxybiphenyl.

2c) einer der Summe aus 2a) und2 b) entsprechenden molaren Menge an wiederkehrenden Einheiten der Formel

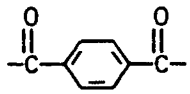

Als Ausgangsverbindung verwendet man Terephthalsäure.

2d) mindestens 10 Mol.% wiederkehrenden Einheiten der Formel

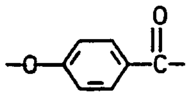

Als Ausgangsverbindung verwendet man p-Hydroxybenzoesäure.

In bevorzugten Polyestern dieses Typs ist ein Teil der Einheiten b) ersetzt durch

2e) wiederkehrende Einheiten der Formel

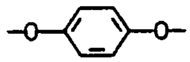

(eine geeignete Ausgangsverbindung ist Hydrochinon) und/oder

2f) wiederkehrende Einheiten der Formel

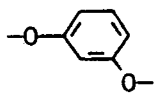

(eine geeignete Ausgangsverbindung ist Resorcin) und/oder

2g) wiederkehrende Einheiten der Formel

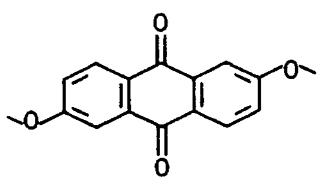

(eine geeignete Ausgangsverbindung ist 2,6-Dihydroxyanthrachinon) und/oder

2h) wiederkehrenden Einheiten der Formel

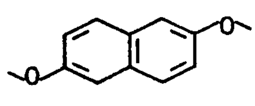

Als geeignete Ausgangsverbindung wird 2,6-Dihydroxynaphthalin verwendet.

In derartigen Polyestern sind die Einheiten 2e), 2f) und/oder 2g) vorteilhaft in einer Menge von 5 bis 12 Mol.% enthalten. Ferner hat es sich als vorteilhaft erwiesen, wenn der molare Anteil an Einheiten 2a) 15 bis 25 Mol.% beträgt. In besonders vorteilhaften Polyestern beträgt der molare Anteil der Summe von 2a) sowie eine oder mehrere Einheiten 2e), 2f) und 2g) 25 bis 35 Mol.%.

Es hat sich als vorteilhaft erwiesen, wenn ein Teil der Einheiten 2c durch Einheiten der Formel

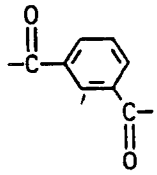

ersetzt wird.

Als geeignete Ausgangsverbindung wird Isophthalsäure verwendet.

Es versteht sich, daß auf die Summe der Hydroxyverbindungen jeweils vorteilhaft die äquivalente Menge Terephthalsäure und/oder Isophthalsäure verwendet wird.

Polyester vom Typ 2 werden in der EP-A 226 078 beschrieben.

3. Thermotrop mesomorphe Polyetherester aus

3a) mindestens 10 Mol.% wiederkehrenden Einheiten der Formel

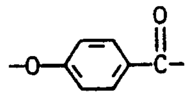

als Ausgangsstoff wird z.B. p-Hydroxybenzoesäure verwendet,

3b) einer Summe aus c) und d) entsprechend e molaren Menge an wiederkehrenden Einheiten der Formel

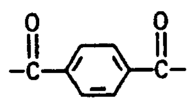

wobei als bevorzugte Ausgangsverbindung Terephthalsäure verwendet wird,

3c) 5 bis 20 Mol.% wiederkehrende Einheiten der Formel

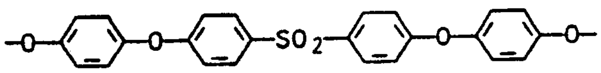 ,

als Ausgangsverbindung wird z.B. 4,4'-Di(p-hydroxyphenoxy)-diphenylsulfon verwendet und

3d) 10 bis 30 Mol.% wiederkehrenden Einheiten der Formel

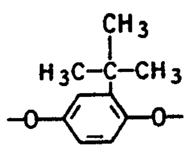 ,

als Ausgangsverbindung wird vorteilhaft tert.-Butylhydrochinon verwendet.

Bevorzugte Polyetherester dieses Typs enthalten neben der Komponente 3a), 10 bis 15 Mol.% Komponente 3c), 15 bis 25 Mol.% Komponente 3d) sowie eine der Summe aus 3c) und 3d) äquivalente molare Menge der Komponente 3b).

Ein Teil der Einheiten 3d) kann durch

3e) wiederkehrende Einheiten der Formel

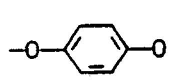 ,

als Ausgangsverbindung wird z.B. Hydrochinon verwendet, und/oder

3f) wiederkehrende Einheiten der Formel

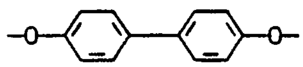,

als geeignete Ausgangsverbindung wird bevorzugt 4,4′-Dihydroxydiphenyl verwendet und/oder
3g) wiederkehrende Einheiten der Formel

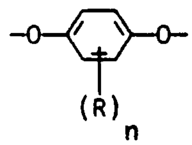

in R jeweils einen Methylrest, Phenylrest oder ein Chloratom bezeichnet und n 1, 2 oder 3 bedeutet, ersetzt werden.

Geeignete Ausgangsverbindungen sind Methylhydrochinon, Trimethylhydrochinon, Phenylhydrochinon und Chlorhydrochinon.

Vorteilhaft enthalten solche vollaromatischen Polyetherester die wiederkehrenden Einheiten 3e) und/oder 3f) in einer Menge von 5 bis 10 Mol.%. In einer anderen bevorzugten Zusammensetzung enthalten die vollaromatischen Polyetherester die Komponente 3g) in einer Menge von 5 bis 20 Mol.%.

Derartige Produkte sind Gegenstand der EP-A 225 539.

4. Polyestercarbamide, aufgebaut aus
4a) mindestens 20 Mol.% wiederkehrenden Einheiten der Formel

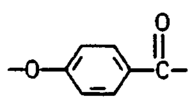,

eine geeignete Ausgangsverbindung ist z.B. p-Hydroxybenzoesäure;
4b) 5 bis 15 Mol.% wiederkehrenden Einheiten der Formel

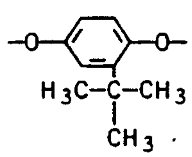,

eine geeignete Ausgangsverbindung ist beispielsweise t-Butylhydrochinon;
4c) 5 bis 15 Mol.% wiederkehrenden Einheiten der Formel

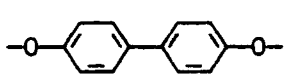,

eine geeignete Ausgangsverbindung ist z.B. 4,4′-Dihydroxydiphenyl;
4d) 1 bis 10 Mol.% wiederkehrenden Einheiten der Formel

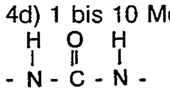,

eine bevorzugte Ausgangsverbindung ist Harnstoff;
4e) einer der Summe aus 4b), 4c) und 4d) entsprechenden molaren Menge an wiederkehrenden Einheiten der Formel

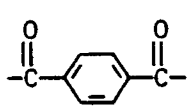,

eine geeignete Ausgangsverbindung ist beispielsweise Terephthalsäure.

Ein Teil der wiederkehrenden Einheiten 4b) kann durch

4f) wiederkehrende Einheiten der Formel

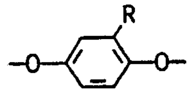

ersetzt werden, in der R einen $C_1$- bis $C_4$-Alkylrest, ausgenommen tert.-Butyl, ein Halogenatom oder einen Phenylrest, der gegebenenfalls Methylgruppen als Substituenten enthalten kann, bedeutet.

Geeignete Ausgangsverbindungen sind beispielsweise Methylhydrochinon, Ethylhydrochinon, Isopropylhydrochinon, Chlorhydrochinon oder Phenylhydrochinon.

Vorteilhaft beträgt der Gehalt an wiederkehrenden Einheiten 4f) 2 bis 10 Mol.%.

Es hat sich ferner als vorteilhaft erwiesen, wenn in den Polyestercarbamiden der molare Anteil der Summe der Komponenten 4b) und 4d) 15 bis 20 Mol.% beträgt.

In anderen Polyestercarbamiden ist ein Teil der Komponente 4c) ersetzt durch mindestens eine der wiederkehrenden Einheiten der Formeln

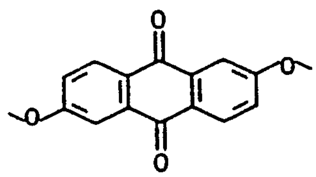

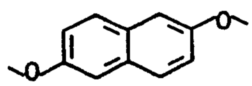

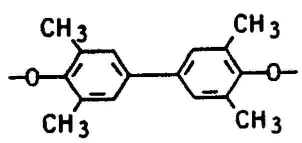

Geeignete Ausgangsverbindungen für diese wiederkehrenden Einheiten der Formeln sind 2,6-Dihydroxyanthrachinon, 2,6-Dihydroxynaphthalin und 3,3′,5,5′-Tetramethyl-4,4′-dihydroxybiphenyl. Vorteilhaft beträgt der Anteil dieser wiederkehrenden Einheiten der Formeln insgesamt 2 bis 10 Mol.%.

Produkte dieses Typs werden in der EP-A 230 551 beschrieben.

5. Polyester aus

5a) mindestens 10 Mol.% wiederkehrenden Einheiten der Formel

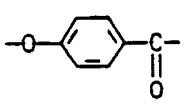 ,

wobei als Ausgangsverbindung 4-Hydroxibenzosäure verwendet wird;

5b) einer der Summe aus den Komponenten 5c) und 5d) äquivalenten molaren Menge an wiederkehrenden Einheiten der Formel

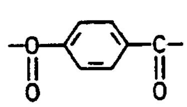 ,

als Ausgangsverbindung wird vorteilhaft Terephthalsäure verwendet;

5c) 3 bis 20 Mol.% wiederkehrenden Einheiten der Formeln

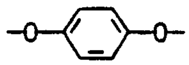

,

als Ausgangsverbindung wird z.B. Hydrochinon verwendet;

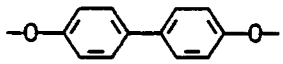

als Ausgangsverbindung wird z.B. 4,4′-Dihydroxibiphenyl verwendet;
5d) 5 bis 30 Mol.% wiederkehrenden Einheiten der Formel

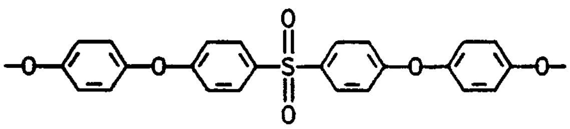

wobei als Ausgangsverbindung vorteilhaft 4,4′-Di-(p-hydroxiphenoxy)-diphenylsulfon verwendet wird.

Bevorzugte Polyetherester sind aufgebaut aus mindestens 20 Mol.%, vorteilhaft bis zu 50 Mol.% der Komponente 5a), 10 bis 25 Mol.% der Komponente 5d), 5 bis 15 Mol.% der Komponente 5c) und einer der Summe aus 5c und 5d) entsprechenden molaren Menge der Komponente 5 b). Vorteilhaft enthalten die Polyetherester als Komponente 5c) 5 bis 15 Mol.% wiederkehrende Einheiten die sich von Hydrochinon oder einer Mischung aus Hydrochinon und Dihydroxydiphenyl ableiten.

Polyetherester des vorstehend genannten Typs sind in der EP-A 226 847 beschrieben.

6. Polyesteramide aus
6a) 5 bis 35 Mol.% wiederkehrenden Einheiten der Formel

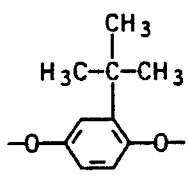

6b) 5 bis 15 Mol.% wiederkehrenden Einheiten der Formel

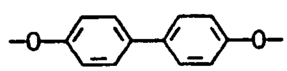

6c) einer der Summe der Komponenten 6a) und 6b) und gegebenenfalls 6e), entsprechenden molare Menge an wiederkehrenden Einheiten der Formel

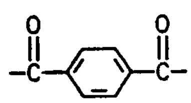

6d) mindestens 10 Mol.-% wiederkehrenden Einheiten der Formel

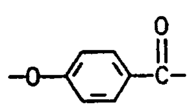

6e) 2 bis 15 Mol.% wiederkehrenden Einheiten der Formeln

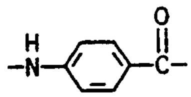

oder

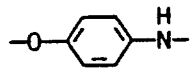

wobei die wiederkehrenden Einheiten der letzten Formel zum Teil ersetzt sein können durch wiederkehrende Einheiten der Formel

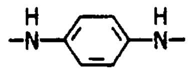

mit der Maßgabe, daß die Summe der molaren Anteile der Komponenten 6a), 6b), 6c), 6d) und 6e) jeweils 100 Mol.% ergibt.

Die wiederkehrenden Einheiten 6a) bis 6e) leiten sich vorzugsweise von folgenden Ausgangsverbindungen ab:

6a) t-Butylhydrochinon
6b) 4,4′-Dihydroxydiphenyl
6c) Terephthalsäure
6d) p-Hydroxybenzoesäure
6e) p-Aminobenzoesäure, p-Aminophenol und 1,4-Diaminobenzol.

Polyesteramide mit diesen Struktureinheiten werden in der EP-A 230 545 beschrieben.

7. Polyesteramide, aufgebaut aus

7a) 3 bis 30 Mol.% wiederkehrenden Einheiten der Formel

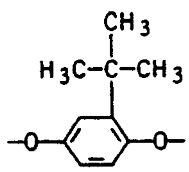

7b) 3 bis 30 Mol.% wiederkehrenden Einheiten der Formeln

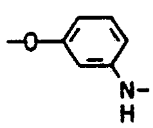

,

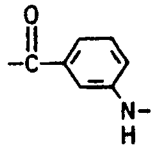

, ,

7c) 2 bis 25 Mol.% mindestens einer der wiederkehrenden Einheiten der Formeln

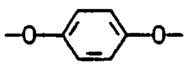 ,

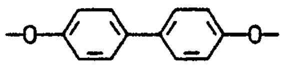 und

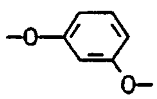 ,

7d) einer der Summe aus den Komponenten 7a), 7b) und 7c), ausgenommen der zweiten wiederkehrenden Einheiten der Formel 7b) entsprechenden molaren Menge an wiederkehrenden Einheiten der Formel

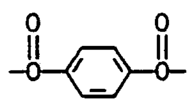 ,

wobei gegebenenfalls ein Teil dieser wiederkehrenden Einheiten durch solche der Formel

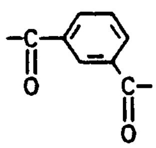 ,

ersetzt sein kann,
7e) gegebenenfalls 5 bis 25 Mol.% wiederkehrenden Einheiten der Formel

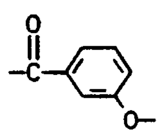 ,

,

7f) wiederkehrenden Einheiten der Formel

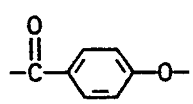 ,

wobei sich die molaren Anteile der Komponenten 7a) bis 7f) jeweils auf 100 Mol.% ergänzen.

Die Einheiten 7a) bis 7f) leiten sich vorzugsweise ab von
7a): t-Butylhydrochinon
7b): m-Aminophenol oder m-Aminobenzoesäure,
7c): Hydrochinon, 4,4′-Dihydroxidiphenyl oder Resorcin,
7d): Terephthalsäure oder Isophthalsäure
7e): m-Hydroxybenzoesäure und
7f): p-Hydroxybenzoesäure.

Bevorzugte Polyesteramide enthalten 5 bis 20 Mol.% wiederkehrende Einheiten 7a), 5 bis 20 Mol.% wiederkehrende Einheiten der Formel 7b), die sich vom m-Aminophenol ableiten und 5 bis 20 Mol.% der Formel 7c), insbesondere solche Einheiten 7c) die sich von Hydrochinon ableiten, sowie die entsprechende Menge an wiederkehrenden Einheiten der Formeln 7d) und außerdem wiederkehrende Einheiten 7f).

Andere bevorzugte Polyesteramide enthalten 5 bis 20 Mol.% Einheiten 7a), 5 bis 20 Mol.% Einheiten 7b), die sich von m-Aminobenzoesäure ableiten sowie 5 bis 20 Mol.% wiederkehrende Einheiten 7c), die sich von Hydrochinon und 4,4′-Dihydroxydiphenyl ableiten, sowie die entsprechende Menge an wiederkehrenden Einheiten der Formeln 7d) und außerdem wiederkehrende Einheiten 7f).

Weitere bevorzugte Polyesteramide enthalten 5 bis 20 Mol.% Einheiten 7a), 5 bis 20 Mol.% Einheiten 7b), die sich von m-Aminobenzoesäure ableiten und 5 bis 20 Mol.% Einheiten 7c), die sich von Hydrochinon ableiten, sowie die entsprechende Menge an wiederkehrenden Einheiten der Formeln 7d) und außerdem wiederkehrende Einheiten 7f).

Polyesteramide des Typs 7 werden in der EP-A 230 546 beschrieben.

8. Polyesteramide, aufgebaut aus

8a) 3 bis 30 Mol.%, insbesondere 5 bis 25 Mol.% wiederkehrenden Einheiten der Formel

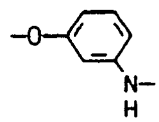 ,

eine geeignete Ausgangsverbindung ist m-Aminophenol;

8b) 3 bis 25 Mol.%, vorzugsweise 5 bis 20 Mol.% mindestens eine der wiederkehrenden Einheiten der Formeln

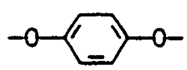 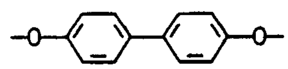 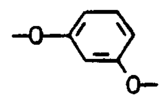 ,

geeignete Ausgangsverbindungen sind beispielsweise Hydrochinon, 4,4′-Dihydroxibiphenyl und Resorcin. Besonders bevorzugt sind wiederkehrende Einheiten die sich von Hydrochinon oder 4,4′-Dihydroxydiphenyl oder deren Mischungen ableiten,

8c) einer der Summe der Komponenten 8a) und 8b) entsprechenden molaren Menge an wiederkehrenden Einheiten der Formeln

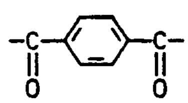 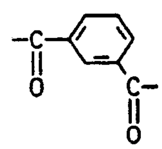 ,

geeignete Ausgangsverbindungen sind beispielsweise Terephthalsäure und Isophthalsäure.

8d) wiederkehrenden Einheiten, die sich von p-Hydroxibenzoesäure ableiten, vorteilhaft in einer Menge von mindestens 10 Mol.%, insbesondere mindestens 20 Mol.%;

8e) gegebenenfalls 5 bis 25 Mol.%, insbesondere 5 bis 15 Mol.% wiederkehrenden Einheiten der Formel

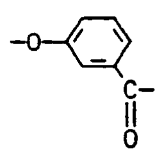 ,

eine geeignete Ausgangsverbindung ist beispielsweise m-Hydroxibenzoesäure.

Polyesteramide mit diesen Einheiten werden in der EP-A 226 080 beschrieben.

9. Polyesterimide aufgebaut aus

9a) 5 bis 35 Mol.% insbesondere 7 bis 31 Mol.% wiederkehrenden Einheiten der Formeln

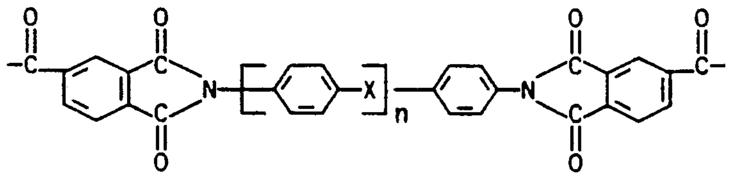

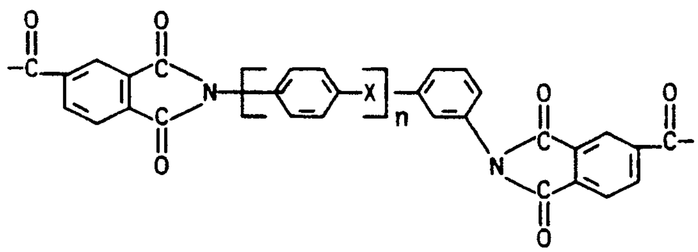

X und n können jeweils gleich oder verschieden sein. X steht für -O-, -S-, $-SO_2-$, -CO-, $-CH_2-$, $-C(CH_3)_2-$, insbesondere für -O-, $-SO_2-$, $-CH_2-$ und n bedeutet 0 oder 1, insbesondere 1. Geeignete Ausgangsverbindungen sind beispielsweise
4,4'-Di-[(4-carboxy)-N-phthalimido]diphenylether,
4,4'-Di-[(4-carboxy)-N-phthalimido]diphenylmethan,
4,4'-Di-[(4-carboxy)-N-phthalimido]diphenylsulfon,
4,4'-Di-[(4-carboxy)-N-phthalimido]diphenylsulfid,
4,4'-Di-[(4-carboxy)-N-phthalimido]diphenylketon,
3,4'- bzw. 3,3'-Di-[(4-carboxy)-N-phthalimido]diphenylether,
3,4'- bzw. 3,3'-Di-[(4-carboxy)-N-phthalimido]diphenylsulfid,
3,4'- bzw. 3,3'-Di-[(4-carboxy)-N-phthalimido]diphenylsulfon,
3,4'- bzw. 3,3'-Di-[(4-carboxy)-N-phthalimido]diphenylketon,
3,4'- bzw. 3,3'-Di-[(4-carboxy)-N-phthalimido]diphenylmethan;
(Verfahren zur Herstellung derartiger Verbindungen sind in Polymer Sciene (A-1), Vol. 7, S. 320-332 beschrieben).
9b) 0 bis 30 Mol.%, insbesondere 0 bis 20 Mol.% wiederkehrenden Einheiten der Formeln

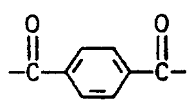 oder 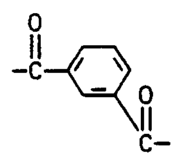 ,

geeignete Ausgangsverbindungen für diese Einheiten sind Terephthalsäure und Isophthalsäure;
9c) einer der Summe aus den Komponenten 9a) und 9b) entsprechenden molaren Menge mindestens einer der wiederkehrenden Einheiten der folgenden Formeln

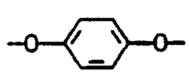 , 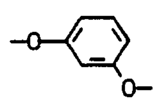 ,

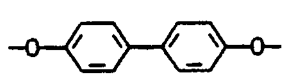 oder

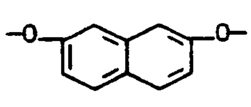 ,

geeignete Ausgangsverbindungen sind beispielsweise Hydrochinon, Resorcin, 4,4'-Dihydroxybiphenyl und

2,7-Dihydroxynaphthalin;

9d) mindestens 10 Mol.%, insbesondere mindestens 20 Mol.% wiederkehrenden Einheiten der Formel

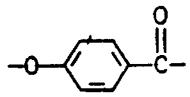

,

eine geeignete Ausgangsverbindung ist beispielsweise p-Hydroxybenzoesäure;

9e) gegebenenfalls 5 bis 25, insbesondere 10 bis 20 Mol.% wiederkehrenden Einheiten der Formel

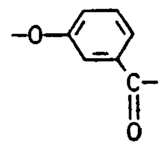

,

eine geeignete Ausgangsverbindung ist beispielsweise m-Hydroxybenzoesäure.

Bevorzugte Polyesterimide enthalten als Komponente 9c) 10 bis 31 Mol.% wiederkehrende Einheiten, die sich von Hydrochinon ableiten, und/oder 5 bis 25 Mol.% wiederkehrende Einheiten, die sich von den anderen unter 9c) aufgeführten Ausgangsverbindungen ableiten. Besonders bevorzugt als Komponente 9c) sind wiederkehrende Einheiten, die sich von Hydrochinon, Resorcin und/oder 4,4′-Dihydroxydiphenyl sowie Mischungen derselben, ableiten.

Polyesterimide des vorstehend genannten Typs werden in der EP-A 227 947 beschrieben.

10. Polyesteramidimide aufgebaut aus

10a) 5 bis 35 Mol.%, insbesondere 7 bis 25 Mol.% wiederkehrenden Einheiten der Formeln

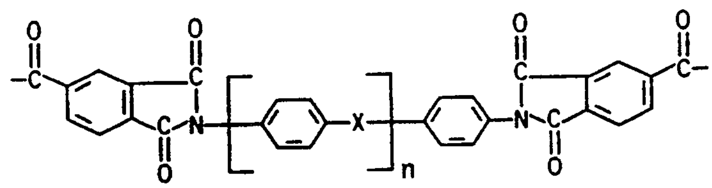

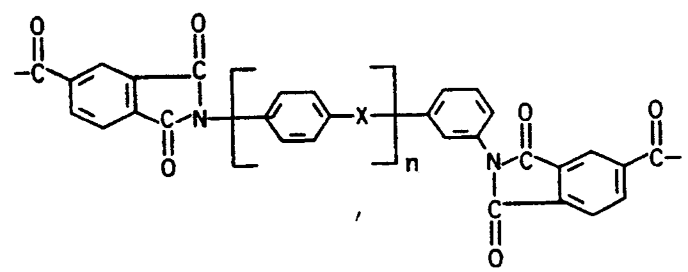

,

,

wobei geeignete Ausgangsverbindungen die gleichen wie die vorstehend für Einheiten 9a) der Polyesterimide 9 genannten Verbindungen sind;

Verbindungen der Formel I und II sind beispielsweise erhältlich nach Polym. Sci. (A-1), 7, 320-332 (1969)

10b) 0 bis 30 Mol.%, insbesondere 0 bis 20 Mol.% wiederkehrenden Einheiten der Formeln

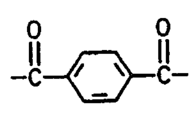

und/oder

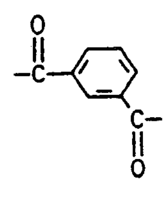

,

wobei beispielsweise Terephthalsäure und Isophthalsäure geeignete Ausgangsverbindungen sind;

10c) einer der Summe der Komponenten 10a) und 10b) entsprechenden molaren Menge mindestens einer der wiederkehrenden Einheiten der folgenden Formeln

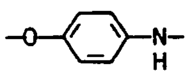 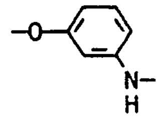 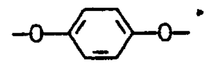

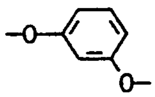 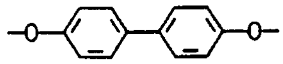

wobei als Ausgangsverbindungen p-Aminophenol, m-Aminophenol, Hydrochinon, Resorcin und 4,4′-Dihydroxidiphenyl zu nennen sind.
10d) gegebenenfalls 5 bis 25 Mol.%, insbesondere 5 bis 20 Mol.% mindestens einer der wiederkehrenden Einheiten der Formeln

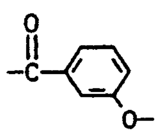 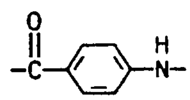 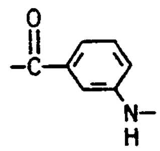

wobei geeignete Ausgangsverbindungen sind m-Hydroxibenzoesäure p-Aminobenzoesäure und m-Aminobenzoesäure sind;
10e) wiederkehrenden Einheiten, insbesondere in einer Menge von mindestens 10 Mol.%, der Formel

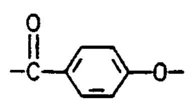

wobei p-Hydroxibenzoesäure als geeignete Ausgangsverbindung zu nennen ist.

Es versteht sich, daß sich die Summe der Molprozente aus der Komponenten 10a) bis 10e) jeweils 100 Mol.% ergibt.

Vorteilhaft enthalten die erfindungsgemäßen Polyesteramidimide als Komponente 10c) 5 bis 20 Mol.% wiederkehrende Einheiten, die sich von m-Aminophenol ableiten, und/oder 2 bis 8 Mol.% wiederkehrende Einheiten die sich von p-Aminophenol und/oder 5 bis 20 Mol.% mindestens einer der wiederkehrenden Einheiten, die sich von Hydrochinon, Resorcin und 4,4′-Dihydroxydiphenyl ableiten.

Besonders bevorzugt sind Polyesteramidimide, die als Komponente 10c) 5 bis 20 Mol.% wiederkehrende Einheiten, die sich von m-Aminophenol sowie 5 bis 15 Mol.% wiederkehrende Einheiten die sich von Hydrochinon und ggf. 5 bis 15 Mol.% wiederkehrende Einheiten , die sich von 4,4′-Dihydroxydiphenyl ableiten, enthalten.

Polymere des Typs 10 werden in der EP-A 230 547 beschrieben.

11. Polyesterimide aufgebaut aus

11a) 5 bis 35 Mol.%, insbesondere 7 bis 25 Mol.%, wiederkehrenden Einheiten der Formeln

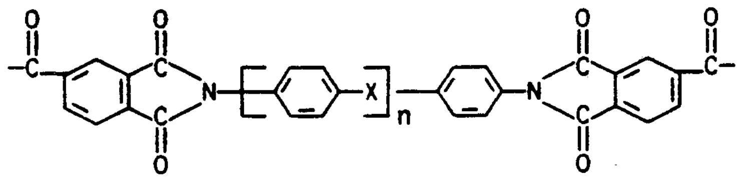

,

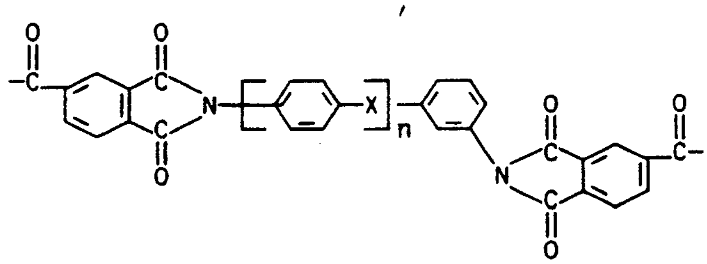

in denen x gleich oder verschieden sein kann und jeweils -O-, -S-, $-SO_2-$, -CO-, $-CH_2$, $-C(CH_3)_2-$ bedeutet und n für 0 oder 1 steht;
Geeignete Ausgangsverbindungen sind bei Polymeren des Typs 9 für die Einheiten 9a) aufgeführt;
Die Herstellung solcher Ausgangsverbindungen wird beispielsweise in J. Polym. Sci. (A-1), 7, 320-332 (1969) beschrieben;
11b) 0 bis 30 Mol.%, insbesondere 0 bis 20 Mol.% wiederkehrende Einheiten der Formeln

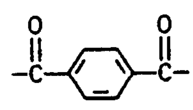 und/oder 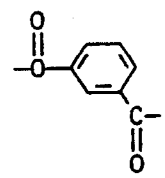 ,

wobei Terephthalsäure und Isophthalsäure als Ausgangsverbindungen zu nennen sind;
11c) einer der Summe aus den Komponenten 11a) und 11b) entsprechenden molaren Menge an aromatischen Dihydroxyverbindungen, nämlich
3 bis 35 Mol.% wiederkehrenden Einheiten der Formel

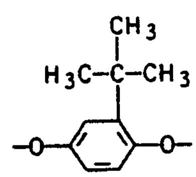

wobei als geeignete Ausgangsverbindung tert.-Butylhydrochinon verwendet werden kann,
sowie mindestens eine der folgenden wiederkehrenden Einheiten in einer Menge von 2 bis 25 Mol.%

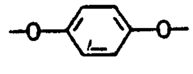 ,

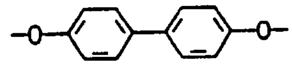

wobei als Ausgangsverbindungen Hydrochinon und 4,4'-Dihydroxydiphenyl zu nennen sind,
sowie 0 bis 25 Mol.% wiederkehrenden Einheiten der Formel

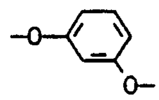

wobei als geeignete Ausgangsverbindung Resorcin zu nennen ist.

11d) gegebenenfalls 5 bis 25 Mol.%, insbesondere 5 bis 20 Mol.% wiederkehrenden Einheiten der Formel

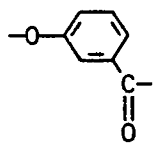 ,

eine geeignete Ausgangsverbindung ist beispielsweise m-Hydroxybenzoesäure;

11e) wiederkehrende Einheiten der folgenden Formel, vorteilhaft in einer Mindestmenge von 10 Mol.%

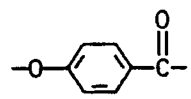 ,

wobei p-Hydroxybenzoesäure als Ausgangsverbindung eingesetzt werden kann.

Es versteht sich, daß die Summe der molaren Anteile der Komponenten 11a), 11b), 11c), 11d) und 11e) jeweils 100 Mol.% ergibt.

Vorteilhafte Polyesterimide enthalten als Komponenten 11c) 5 bis 25 Mol.% von t-Butylhydrochinon abgeleitete wiederkehrende Einheiten sowie 5 bis 20 Mol.% mindestens einer der wiederkehrenden Einheiten, die sich von Hydrochinon, Resorcin oder 4,4′-Dihydroxydiphenyl ableiten.

Weitere bevorzugte Arten von Polyesterimiden des Typs 11 sind in der EP-A 225 537 beschrieben.

12. Polyesteramidimide aufgebaut aus

12a) 5 bis 35 Mol.% wiederkehrenden Einheiten der Formel

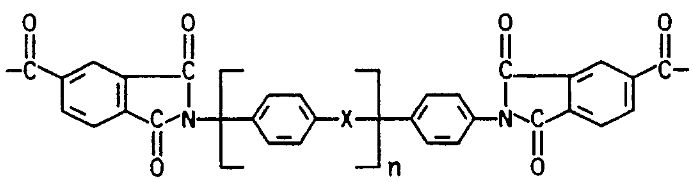

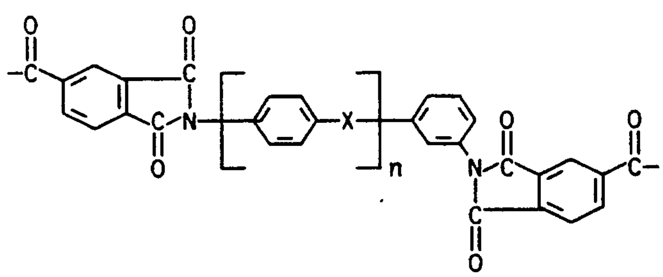

wobei X und n die vorstehend bei den Polymeren 9 für die Einheiten 9a) beschriebene Bedeutung haben;

12b) 0 bis 30 Mol.% wiederkehrenden Einheiten, die sich von Terephthalsäure und/oder Isophthalsäure ableiten;

12c) einer der Summe der Komponenten 12a) und 12b) entsprechenden molaren Menge mindestens einer der folgenden wiederkehrenden Einheiten

12c1) 3 bis 35 Mol.% Einheiten der Formel

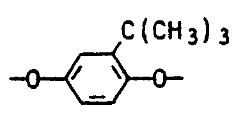 ,

12c2) 0 bis 10 Mol.% Einheiten der Formel

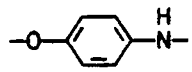 ,

12c3) 2 bis 25 Mol.% Einheiten der Formel

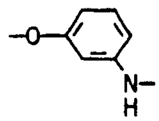 ,

12c4) 2 bis 20 Mol.% Einheiten der Formel

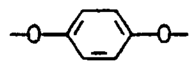 ,

12c5) 0 bis 20 Mol.% Einheiten der Formel

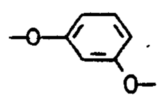 ,

und
12c6) 2 bis 20 Mol.% Einheiten der Formel

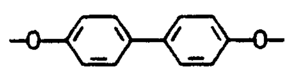

wobei geeignete Ausgangsverbindungen t-Butylhydrochinon, p-Aminophenol, m-Aminophenol, Hydrochinon, Resorcin und 4,4′-Dihydroxydiphenyl sind.
12d) mindestens 10 Mol.% wiederkehrenden Einheiten der Formel

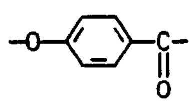

wobei als Ausgangsverbindung p-Hydroxibenzoesäure zu nennen ist; und
12e) 0 bis 25 Mol.% mindestens einer der wiederkehrenden Einheiten der Formel

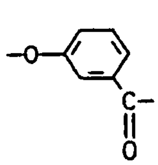 , 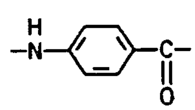 oder 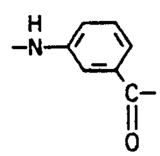

wobei diese Einheiten sich von m-Hydroxybenzoesäure, p-Aminobenzoesäure und m-Aminobenzoesäure ableiten.

Polyesteramidimide vom Typ 12 werden in der EP-A 225 529 beschrieben.

13. Polyetheresterimide aufgebaut aus
13a) 5 bis 35 Mol.% wiederkehrenden Einheiten der Formel

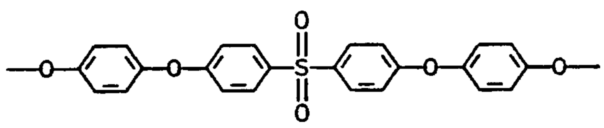 ,

wobei 4,4′-Di-(p-hydroxyphenoxy)-diphenylsulfon als geeignete Ausgangsverbindung zu nennen ist;
13b) 5 bis 35 Mol.% wiederkehrenden Einheiten der Formeln

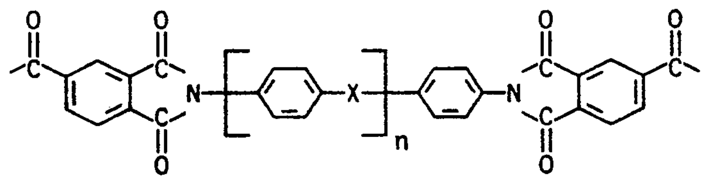

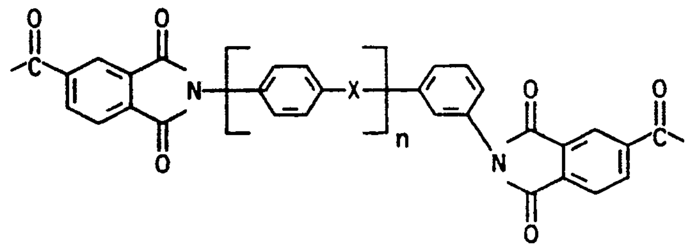

in der X eine chemische Bindung -O-, -S-, $-SO_2-$, -CO-, $-CH_2-$ oder $=C(CH_3)_2$ bezeichnet und n für 0 oder 1 steht. Geeignete Ausgangsverbindungen sind bei Polymeren des Typs 9 unter den Einheiten 9a) aufgeführt.
13c) 15 bis 30 Mol.% wiederkehrenden Einheiten, die sich von Terephthalsäure ableiten,
13d) einer der Summe aus den Komponenten 13b) plus 13c) minus 13a) entsprechenden molaren Menge an wiederkehrenden Einheiten der Formeln

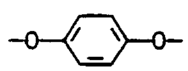 und/oder 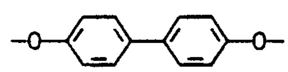 ,

die sich von Hydrochinon und/oder 4,4′-Dihydroxydiphenyl ableiten,
13e) wiederkehrenden Einheiten, die sich von p-Hydroxybenzoesäure ableiten.

Es versteht sich, daß sich die Molprozente der Komponenten 13a) bis 13e) jeweils auf 100 Mol.% ergänzen.

Bevorzugte Polyetheresterimide sind aufgebaut aus
10 bis 30 Mol.% wiederkehrenden Einheiten 13a)
10 bis 30 Mol.% wiederkehrenden Einheiten 13b)
15 bis 30 Mol.% wiederkehrenden Einheiten 13c)
einer der Summe aus 13b) plus 13c) minus 13a) entsprechenden molaren Menge an wiederkehrenden Einheiten 13d) sowie
wiederkehrenden Einheiten der Formel 13e).

Besonders bewährt haben sich Polyetheresterimide die aufgebaut sind aus
15 bis 25 Mol.% wiederkehrenden Einheiten 13a),
15 bis 25 Mol.% wiederkehrenden Einheiten 13b),
20 bis 30 Mol.% wiederkehrenden Einheiten 13c),
einer der Summe aus 13b) plus 13c) minus 13a) entsprechenden molaren Menge an wiederkehrenden Einheiten 13d), insbesondere solche, die sich von Hydrochinon ableiten, und
wiederkehrenden Einheiten 13e), vorteilhaft in einer Menge von mindestens 10 Mol.%.

Derartige thermotrope Polymere werden in der EP-A 231 642 beschrieben.

14. Mesomorphe Polykondensate aufgebaut aus
14a) mindestens 10 Mol.%, vorteilhaft mindestens 20 Mol.% wiederkehrenden Einheiten, die sich von p-Hydroxybenzoesäure ableiten,

14b) 5 bis 30 Mol.%, vorteilhaft 10 bis 25 Mol.% wiederkehrenden Einheiten, die sich von Methylhydrochinon ableiten,
14c) 0 bis 20 Mol.%, insbesondere 2 bis 18 Mol.% mindestens einer der wiederkehrenden Einheiten der Formeln

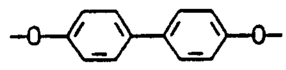

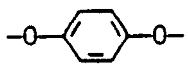

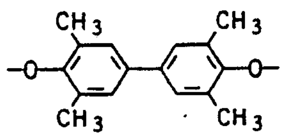

wobei als Ausgangsverbindungen 4,4′-Dihydroxydiphenyl, Hydrochinon und 3,3′,5,5′-Tetramethyl-4,4′-dihydroxydiphenyl zu nennen sind,
14d) 0 bis 20 Mol.%, vorteilhaft 2 bis 15 Mol.% mindestens einer der wiederkehrenden Einheiten der folgenden Formeln

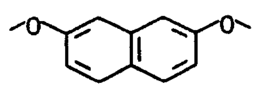 ,

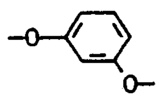 ,

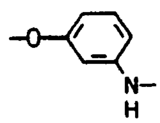 ,

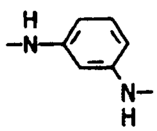 ,

wobei als Ausgangsverbindungen 2,7-Dihydroxynaphthalin, Resorcin, m-Aminophenol und m-Phenylendiamin zu nennen sind,
14e) einer der Summe der Komponenten 14b), 14c) und 14d) entsprechenden molaren Menge an wiederkehrenden Einheiten, die sich von Terephthalsäure ableiten,
wobei die Summe der Molprozente der Komponenten 14a), 14b), 14c), 14d) und 14e) jeweils 100 Mol.% ergibt.

Es ist auch möglich, daß ein Teil, z.B. bis zu 50 % der erforderlichen molaren Menge an Einheiten, die sich von Terephthalsäure ableiten, ersetzt sind durch wiederkehrende Einheiten die sich von Isophthalsäure ableiten.

Ferner ist es möglich, einen Teil der Komponenten 14a), z.B. bis zu einer Menge von 15 Mol.% zu ersetzen durch mindestens eine der wiederkehrenden Einheiten der folgenden Formeln

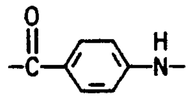

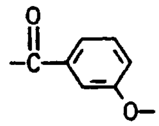

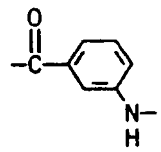

Geeignete Ausgangsverbindungen sind p-Aminobenzoesäure, m-Hydroxybenzoesäure und m-Aminobenzoesäure.

Derartige Produkte werden in der EP-A-230 550 beschrieben.

15. Flüssig-kristalline Carbamidgruppen enthaltende Polykondensate, aufgebaut aus

15a) aromatischen Hydroxy- und/oder Aminocarbonsäuren, in denen die Hydroxy- oder Aminogruppe nicht vicinal zur Carboxylgruppe stehen

15b) 0,1 bis 20 Mol.% Harnstoff

15c) aromatischen Dihydroxy-, Diamino- und/oder Hydroxy-Aminoverbindungen, in denen die Hydroxy- und Aminogruppen nicht vicinal zueinander angeordnet sind

15d) einer der Summe der Komponenten b und c äquivalenten molaren Menge an aromatischen Dicarbonsäuren, in denen die Carboxylgruppen nicht vicinal angeordnet sind.

Bevorzugte Polymere dieses Typs enthalten

15a) mindestens 10 Mol.% mindestens einer der wiederkehrenden Einheiten

$-O-R^1-\overset{O}{\overset{\|}{C}}-$ oder

$-\overset{H}{N}-R^2-\overset{O}{\overset{\|}{C}}-$

in denen $R^1$ und $R^2$ gleich oder verschieden sein können und jeweils einen 1,4-Phenylen-, 1,3-Phenylenrest, 2,7-Naphthylen- oder 2,6-Naphthylenrest bezeichnen

15b) 0,1 bis 20 Mol.% wiederkehrende Einheiten der Formel

$-\overset{H}{N}-\overset{O}{\overset{\|}{C}}-\overset{H}{N}-$

15c) mindestens eine der wiederkehrenden Einheiten der Formel

$-X-R^3-Y-$

in der $R^3$ einen 1,4-Phenylen-, 1,3-Phenylen-, 2,6-Naphthylen-, 2,7-Naphthylen-, 4,4′-Biphenylylen-, 3,4′-Biphenylylen, 2,6-Antrachinonylen-rest bezeichnet, wobei die Reste auch Halogenatome, Alkylreste mit 1 bis 4 Kohlenstoffatomen oder einen Phenylrest als Substituenten haben können und darüber hinaus $R^3$ auch für den Rest

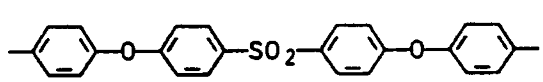

steht und X und Y gleich oder verschieden sein können und jeweils für ein Sauerstoffatom oder den Rest -NH- stehen,

15d) eine der Summe aus den Komponenten 15b) und 15c) äquivalenten Menge an wiederkehrenden Einheiten der Formel

$-\overset{O}{\overset{\|}{C}}-R^4-\overset{O}{\overset{\|}{C}}-$

in der $R^4$ für einen 1,4-Phenylen-, 1,3-Phenylen-rest oder Reste der folgenden Formeln

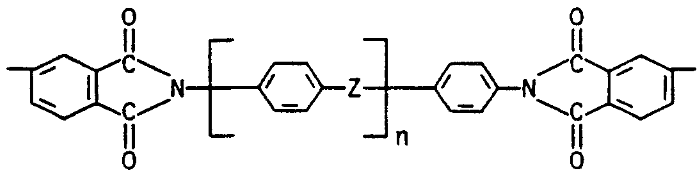

,

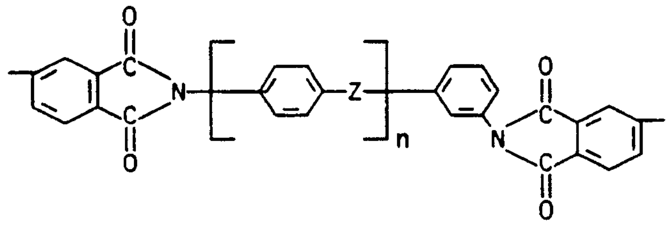

steht, in der Z jeweils für -O-, -S-, $-SO_2-$, -CO-, $-CH_2-$, $=C(CH_3)_2$ steht und n 0 oder 1 bedeutet, oder $R^4$ auch für Reste der Formeln

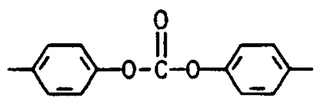

,

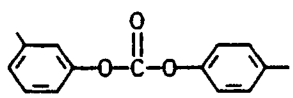

und

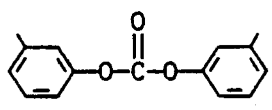

steht.

Bevorzugte Polykondensate dieses Typs sind beispielsweise

15/1. vollaromatische mesomorphe Polyetherestercarbamide, aufgebaut aus

15a) mindestens 10 Mol.% 4-Hydroxy-benzoesäure

15b) 0,5 bis 12 Mol.% Harnstoff

15$c_1$) 3 bis 20 Mol.% Hydrochinon und/oder 4,4′-Dihydroxydiphenyl,

15$c_2$) 5 bis 30 Mol.% 4,4′-Di(p-hydroxyphenoxy)-diphenylsulfon und

15d) einer der Summe der Komponenten 15b) und 15c) entsprechenden äquivalenten molaren Menge an Terephthalsäure.

15/2. Polyestercarbamide, aufgebaut aus

15a) mindestens 10 Mol.% 4-Hydroxy-benzoesäure

15b) 0,1 bis 11 Mol.% Harnstoff

15$c_1$) 10 bis 25 Mol.% 3,3′,5,5′-Tetramethyl-4,4′-dihydroxydiphenyl

15$c_2$) 5 bis 15 Mol.% Hydrochinon

15$c_3$) 5 bis 15 Mol.% 4,4′-Dihydroxydiphenyl und

15d) einer der Summe aus den Komponenten 15b) und 15c) entsprechenden molaren Menge an Terephthalsäure.

15/3. Polyestercarbamide, aufgebaut aus

15a) mindestens 10 Mol.% 4-Hydroxy-benzoesäure

15b) 0,1 bis 7 Mol.% Harnstoff

15$c_1$) 5 bis 35 Mol.% tert.-Butylhydrochinon

15$c_2$) 2 bis 30 Mol.% 4,4′-Dihydroxydiphenyl und

15d) einer der Summe aus 15b) und 15c) entsprechenden molaren Menge an Terephthalsäure.

15/4. Polyesteramidcarbamide, aufgebaut aus

15a) mindestens 10 Mol.% 4-Hydroxy-benzoesäure, die teilweise durch 4-Aminobenzoesäure ersetzt sein kann

15b) 0,4 bis 10 Mol.% Harnstoff

15$c_1$) 3 bis 30 Mol.% tert.-Butylhydrochinon

15$c_2$) 3 bis 30 Mol.% 3-Aminophenol

15$c_3$) 2 bis 25 Mol.% mindestens einer der Verbindungen Hydrochinon, 4,4′-Dihydroxydiphenyl,

Resorcin und

15d) einer der Summe aus den Komponenten 15b) und 15c) entsprechenden molaren Menge an Terephthalsäure, die teilweise durch Isophthalsäure ersetzt sein kann.

15/5. Polyesteramidcarbamide, aufgebaut aus

15a) mindestens 10 Mol.% 4-Hydroxybenzoesäure und gegebenenfalls 5 bis 25 Mol.% 3-Hydroxybenzoesäure

15b) 0,1 bis 7 Mol.% Harnstoff

$15c_1$) 3 bis 30 Mol.% 3-Aminophenol

$15c_2$) 3 bis 25 Mol.% mindestens einer der Verbindungen Hydrochinon, 4,4′-Dihydroxydiphenyl oder Resorcin und

15d) einer der Summe der Komponenten 15b) und 15c) entsprechenden molaren Menge an Terephthalsäure und/oder Isophthalsäure.

15/6. Polyetherestercarbamide, aufgebaut aus

15a) mindestens 10 Mol.% 4-Hydroxybenzoesäure

15b) 0,1 bis 7 Mol.% Harnstoff

$15c_1$) 5 bis 20 Mol.% 4,4′-Di(p-hydroxyphenoxy)-diphenylsulfon

$15c_2$) 10 bis 30 Mol.% tert.-Butylhydrochinon und

15d) einer der Summe der Komponenten 15b) und 15c) äquivalenten molaren Menge an Terephthalsäure.

15/7. Polyetheresterimidcarbamide, aufgebaut aus

15a) mindestens 10 Mol.% 4-Hydroxybenzoesäure

15b) 0,1 bis 5 Mol.% Harnstoff

$15c_1$) 5 bis 35 Mol.% 4,4′-Di(p-hydroxyphenoxy)-diphenylsulfon

$15c_2$) einer Summe aus den Komponenten (d1 + d2) - ($c_1$ + b) entsprechenden molaren Menge an Hydrochinon und/oder 4,4′-Dihydroxydiphenyl und

$15d_1$) 5 bis 35 Mol.% 4,4′-Bis[(4-carboxy)-N-phthalimido]-diphenylether-methan, -sulfon, -sulfid oder -keton und

$15d_2$) 15 bis 30 Mol.% Terephthalsäure.

15/8. Polyestercarbamide, aufgebaut aus

15a) mindestens 20 Mol.% 4-Hydroxibenzoesäure

15b) 1 bis 10 Mol.% Harnstoff

$15c_1$) 5 bis 15 Mol.% tert.-Butylhydrochinon

$15c_2$) 5 bis 15 Mol.% 4,4′-Dihydroxydiphenyl und

15d) einer der Summe der Komponenten 15b) und 15c) entsprechenden molaren Menge an Terephthalsäure.

Es versteht sich, daß sich in den vorgegebenen Polykondensaten die Komponenten jeweils auf 100 Mol.% ergänzen.

Polymere dieser Strukturen werden in der EP-A 231 468 beschrieben.

16. Vollaromatische mesomorphe Polyester die unterhalb von 300° C eine flüssigkristalline fadenbildende Schmelze bilden, aufgebaut im wesentlichen aus

16a) 5 bis 25 Mol% wiederkehrenden Einheiten der Formel

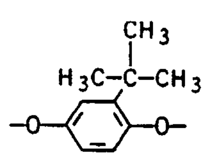

16b) 5 bis 25 Mol% wiederkehrenden Einheiten der Formel

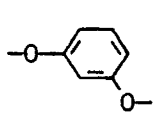

16c) 10 bis 50 Mol% wiederkehrenden Einheiten der Formel

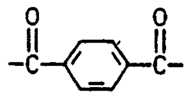

und

16d) mindestens 10 Mol% wiederkehrenden Einheiten der Formel

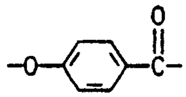

wobei die Summe der molaren Anteile der Komponenten 16a), 16b), 16c) und 16d) jeweils 100 Mol% ergibt und das molare Verhältnis der Komponenten 16a) und 16b) zur Komponente 16c) im Bereich von 0,9:1 bis 1,1:1 liegt.

Als Ausgangsverbindungen für die verschiedenen Einheiten sind t-Butylhydrochinon, Resorcin, Terephthalsäure und p-Hydroxybenzoesäure zu nennen.

17. Thermotrop mesomorphe Polyester aufgebaut aus

17a) mindestens 10 Mol.% Einheiten, die sich von p-Hydroxybenzoesäure ableiten,

17b) 1 bis 25 Mol.% wiederkehrenden Einheiten der Formel

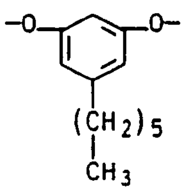

17c) 5 bis 20 Mol.% mindestens einer der wiederkehrenden Einheiten der folgenden Formeln

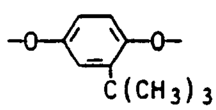

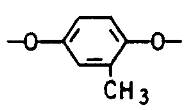

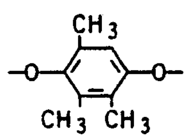

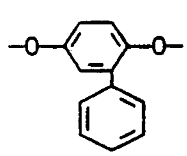

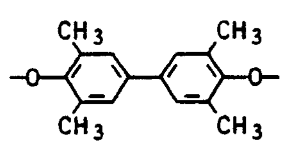

17d) 5 bis 15 Mol.% wiederkehrenden Einheiten der Formel

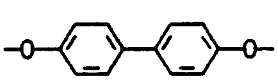

17e) 10 bis 60 mol.% wiederkehrenden Einheiten der Formel

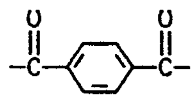

wobei die Summe der molaren Anteile 17a), 17b), 17c) und 17e) jeweils 100 mol.% ergibt und das molare Verhältnis der Komponenten 17b) + 17c) + 17d) zur Komponente 17e) im Bereich von 0,9:1 bis 1,1:1 liegt.

Bevorzugte Ausgangsstoffe sind für wiederkehrende Einheiten der Formel

17b) 3-n-Hexylresorcin,

17c) t-Butylhydrochinon, Methylhydrochinon, Trimethylhydrochinon, Phenylhydrochinon und 3,3′,5,5′-Tetramethyl-4,4′-dihydroxydiphenyl

17d) 4,4′-Dihydroxydiphenyl

17e) Terephthalsäure.

18. Vollaromatische thermotrop mesomorphe Polyester auf der Basis von

18a) 30 bis 60 mol% 4-Hydroxybenzoesäure

18b) 20 bis 35 mol% einer Mischung aus

$b_1$) Terephthalsäure und

$b_2$) Isophthalsäure

wobei das molare Verhältnis von $b_1$ : $b_2$ im Bereich von 1,04:1 bis 19:1, vorzugsweise von 1,5:1 bis 10:1 liegt, und

18c) 20 bis 35 mol% einer Mischung aus

$18c_1$) Hydrochinon

$18c_2$) 4,4′-Dihydroxydiphenyl und

$18c_3$) 0 bis 5 Mol.% einer Dihydroxyverbindung der allgemeinen Formel

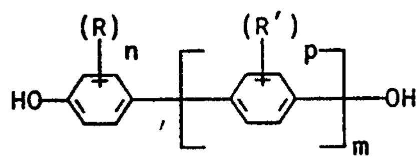

wobei R und R′ Alkylgruppen mit 1 bis 4 C-Atomen, Halogen oder eine Arylgruppe darstellen, n und p den Wert 1, 2 oder 3 und m den Wert 0 oder 1 haben, das molare Verhältnis von $18c_1$) : $18c_2$) im Bereich von 0,1:1 bis 2,67:1, vorzugsweise von 0,5:1 bis 2,33:1 liegt und

das molare Verhältnis von 18b):18c) im Bereich von 0,9:1 bis 1,1:1, vorzugsweise von 0,98:1 bis 1,02:1 liegt.

Die Summe der Komponenten 18a) + 18b) + 18c) ergibt stets 100 mol%.

19. Vollaromatische thermotrop mesomorphe Polyester auf der Basis von

19a) 30 bis 60 mol% 4-Hydroxibenzoesäure

19b) 20 bis 35 mol% einer Mischung aus

$19b_1$) Terephthalsäure und

$19b_2$) Isophtalsäure

wobei das molare Verhältnis von $19b_1$) : $19b_2$) im Bereich von 1,04:1 bis 19:1, vorzugsweise von 1,5:1 bis 10:1 liegt, und

19c) 20 bis 35 mol% einer Mischung aus

$19c_1$) Hydrochinon

$19c_2$) 4,4′-Dihydroxydiphenyl und

$19c_3$) 0,5 bis 5 mol%

$19c_{31}$) 2,7-Dihydroxynaphthalin und/oder

$19c_{32}$) 1,3-Dihydroxybenzol und/oder

$19c_{33}$) einer aromatischen Dihydroxyverbindung der allgemeinen Formel

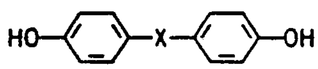

wobei X $-CH_2-$, $-C(CH_3)_2-$, -S-, $-SO_2-$, -O- oder -CO-ist, oder deren kernsubstituierten Chlor-, Brom-, Aryl-

oder $C_1$-$C_8$-Alkyl- oder Alkoxyderivaten, und/oder
$19c_{34}$) einer Verbindung der allgemeinen Formel

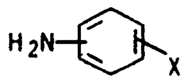

wobei X $-NH_2$ oder -OH ist und sich die Substituenten in meta-oder para-Stellung zueinander befinden, oder deren kernsubstituierten Chlor-, Brom-, Aryl- oder $C_1$-$C_8$-Alkyl- oder Alkoxyderivaten,
wobei das molare Verhältnis von $19c_1$) : $19c_2$) im Bereich von 0,1:1 bis 2,67:1 vorzugsweise von 0,5:1 bis 2,33:1 und das molare Verhältnis von 19b):19c) im Bereich von 0,9:1 bis 1,1:1, vorzugsweise von 0,98:1 bis 1,02:1 liegt.

20. Vollaromatische thermotrop mesomorphe Polyester auf der Basis von
20a) 25 bis 60 mol% einer Mischung aus
$20a_1$) 4-Hydroxibenzoesäure und
$20a_2$) 3-Hydroxibenzoesäure, 4-Aminobenzoesäure und/oder 3-Aminobenzoesäure oder deren kernsubstituierten Chlor, Brom-, $C_1$-$C_8$-Alkyl- oder $C_1$-$C_8$-Alkoxyderivaten
wobei das molare Verhältnis $20a_1$) : $20a_2$) im Bereich von 5:1 bis 41:1, vorzugsweise von 6:1 bis 20:1 liegt,
20b) 20 bis 37,5 mol% einer Mischung aus
$20b_1$) Terephthalsäure und
$20b_2$) Isophthalsäure
wobei das molare Verhältnis von $20b_1$) : $20b_2$) im Bereich von 1,04:1 bis 19:1, vorzugsweise von 1,5:1 bis 10:1 liegt, und
20c) 20 bis 37,5 mol% einer Mischung aus
$20c_1$) Hydrochinon
$20c_2$) 4,4′-Dihydroxydiphenyl
wobei das molare Verhältnis von $20c_1$) : $20c_2$) im Bereich von 0,1:1 bis 2,67:1, vorzugsweise von 0,5:1 bis 2,33:1 und das molare Verhältnis von 20b):20c) im Bereich von 0,9:1 bis 1,1:1, vorzugsweise von 0,98:1 bis 1,02:1 liegt.

21. Vollaromatische thermotrop mesomorphe Polyester auf der Basis von
21a) 30 bis 60 mol% 4-Hydroxibenzoesäure
21b) 20 bis 35 mol% einer Mischung aus
$21b_1$) Terephthalsäure und
$21b_2$) Isophthalsäure
$21b_3$) 0,5 bis 5 mol% einer Dicarbonsäure der allgemeinen Formel

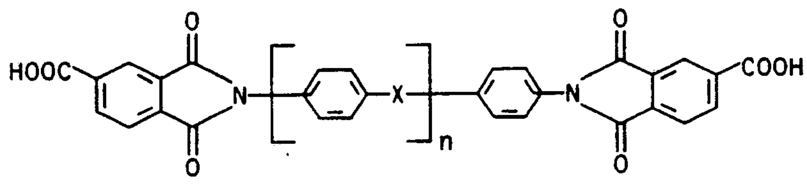

wobei X -O-, -S-, $-SO_2-$, -CO-, $-CH_2-$ oder $-C(CH_3)_2-$ ist, n den Wert 0 oder 1 hat und die beiden Imid-Stickstoffatome in meta-oder para-Stellung zu X stehen, oder deren kernsubstituierten $C_1$-$C_8$-Alkyl-, $C_1$-$C_8$-Alkoxy-, Aryl-, Chlor-oder Bromderivaten und
wobei das molare Verhältnis von $21b_1$) : $21b_2$) im Bereich von 1,04:1 bis 19:1, vorzugsweise von 1,5:1 bis 10:1 liegt, und
21c) 20 bis 35 mol% einer Mischung aus
$21c_1$) Hydrochinon
$21c_2$) 4,4′-Dihydroxydiphenyl
wobei das molare Verhältnis von $21c_1$) : $21c_2$) im Bereich von 0,1:1 bis 2,67:1, vorzugsweise von 0,5:1 bis 2,33:1 und das molare Verhältnis von 21b):21c) im Bereich von 0,9:1 bis 1,1:1, vorzugsweise von 0,98:1 bis 1,02:1 liegt.

Als geeignete Ausgangsverbindungen $21b_3$) sind die für die Polymere des vorstehend beschriebenen Typs 9 unter der Einheit 9a) aufgeführten Verbindungen zu nennen.

22. Thermotrop mesomorphe vollaromatische Polyester aus der Basis von
22a) 30 bis 60 Mol.% 4-Hydroxybenzoesäure,

22b) 20 bis 35 Mol.% Terephthalsäure und

22c) 20 bis 35 Mol.% einer Mischung aus

$22c_1$) Trimethylhydrochinon und

$22c_2$) Resorcin

im molaren Verhältnis $22c_1$):$22c_2$) von 0,4:1 bis 0,7:1, vorzugsweise von 0,45:1 bis 0,65:1, und das molare Verhältnis von 22b):22c) im Bereich 0,9:1 bis 1,1:1, vorzugsweise von 0,98:1 bis 1,02:1 liegt.

23. Vollaromatische thermotrop mesomorphe Polyester auf der Basis von

23a) 3 bis 15 Mol.% Einheiten, die sich von Hydrochinon ableiten,

23b) 5 bis 35 Mol.% Einheiten, insbesondere 25 bis 35 Mol.%, die sich von 2,7-Dihydroxynaphthalin ableiten,

23c) einer der Summe aus 23a) und 23b) äquivalenten molaren Menge an Einheiten, die sich von Terephthalsäure ableiten, und

23d) 10 bis 70 Mol.%, insbesondere 10 bis 39,4 Mol.% Einheiten, die sich von p-Hydroxybenzoesäure ableiten.

Ein Teil der wiederkehrenden Einheiten 23a) und 23b) kann durch eine oder mehrere Einheiten, die sich von

23e) 4,4′-Dihydroxydiphenyl und/oder

23f) 4,4′-Dihydroxydiphenylsulfon und/oder

23g) 2,2-Di-(4-hydroxyphenyl)propan ableiten,

ersetzt werden.

Vorteilhaft beträgt der Gehalt an wiederkehrenden Einheiten 23e), 23f) und/oder 23g) 2 bis 20 Mol.%.

Besonders bevorzugt sind Polyester, wenn der Gehalt an wiederkehrenden Einheiten 23b), sowie einer oder mehrerer wiederkehrender Einheiten 23e), 23f) und/oder 23g) 25 bis 40 Mol.% beträgt.

Produkte dieses Typs werden in der EP-A 139 303 beschrieben.

24. Vollaromatische, thermotrop mesomorphe Polyester, die aufgebaut sind aus

24a) 10 bis 90 Mol.% wiederkehrenden Einheiten der Formel

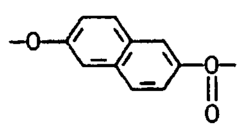

(eine geeignete Ausgangsverbindung ist z.B. 2-Hydroxy-6-carboxy-naphthalin), und

24b) 10 bis 90 Mol.% wiederkehrenden Einheiten der Formel

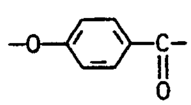

(eine geeignete Ausgangsverbindung ist z.B. p-Hydroxybenzoesäure).

Vorteilhaft enthalten solche vollaromatischen Polyester die wiederkehrenden Einheiten 24a) in einer Menge von 65 bis 85 Mol.% und besonders bevorzugt in Mengen von 70 bis 80 Mol.%.

25. Vollaromatische, thermotrop mesomorphe Polyester, die aufgebaut sind aus

25a) 30 bis 70 Mol.%, insbesondere 40 bis 60 Mol.% wiederkehrenden Einheiten der Formel

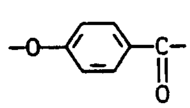

(eine geeignete Ausgangsverbindung ist z.B. p-Hydroxybenzoesäure).

25b) 20 bis 30 Mol.% wiederkehrenden Einheiten der Formel

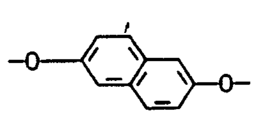

(eine geeignete Ausgangsverbindung ist z.B. 2,6-Dihydroxinaphthalin), und
25c) 20 bis 30 Mol.% wiederkehrenden Einheiten der Formel

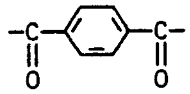

(eine geeignete Ausgangsverbindung ist z.B. Terephthalsäure).

Es versteht sich, daß die wiederkehrenden Einheiten aus Alkyl- oder Alkoxyreste mit 1 bis 4 Kohlenstoffatomen, Halogenatome, Phenylreste oder Kombinationen derselben als Substituenten haben können.

26. Thermotrop mesomorphe Polyester, die aufgebaut sind aus
26a) 20 bis 60 Mol.%, insbesondere 25 bis 45 Mol.% der wiederkehrenden Einheiten der Formel

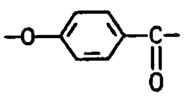

(eine geeignete Ausgangsverbindung ist z.B. p-Hydroxybenzoesäure),
26b) 5 bis 18 Mol.% der wiederkehrenden Einheiten der Formel

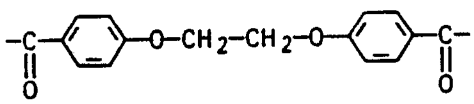

(eine geeignete Ausgangsverbindung ist z.B. 1,2-Di-(4-carboxy-phenoxy)ethan),
26c) 5 bis 35 Mol.% wiederkehrenden Einheiten der Formel

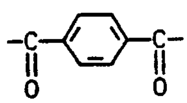

(eine geeignete Ausgangsverbindung ist z.B. Terephthalsäure), und
26d) 20 bis 40 Mol.% wiederkehrenden Einheiten der Formel

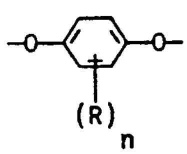

wobei R jeweils einen Methylrest, ein Chlor- oder ein Bromatom, oder Kombinationen derselben bezeichnen und n den Wert 1, 2 oder 3 hat.

Bevorzugte Polyester enthalten 35 bis 45 Mol.% der wiederkehrenden Einheiten 26a), 10 bis 15 Mol.% der wiederkehrenden Einheiten 26b), 15 bis 25 Mol.% der wiederkehrenden Einheiten 26c) und 25 bis 35 Mol.% der wiederkehrenden Einheiten 26d).

Es versteht sich, daß die Summe der Komponenten 26b) und 26c) äquimolar zur Menge der Komponente 26d) ist.

Weiterhin können auch die Komponenten 26a), 26b) und 26c) mit Alkyl- oder Alkoxyresten mit 1 bis 4 Kohlenstoffatomen, Halogenatomen, Phenylresten oder Kombinationen derselben substituiert sein.

27. Thermotrop mesomorphe Polyester die aufgebaut sind aus
27a) 20 bis 40 Mol.%, insbesondere 20 bis 30 Mol.% der wiederkehrenden Einheiten der Formel

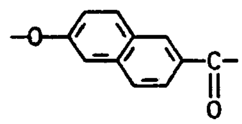

(eine geeignete Ausgangsverbindung ist z.B. 2-Hydroxy-6-naphthalin-carbonsäure),
27b) 10 bis 50 Mol.%, insbesondere 25 bis 40 Mol.% der wiederkehrenden Einheiten der Formel

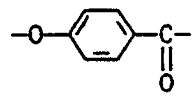

(eine geeignete Ausgangsverbindung ist z.B. p-Hydroxybenzoesäure),
27c) 5 bis 30 Mol.%, insbesondere 15 bis 25 Mol.% an wiederkehrenden Einheiten der Formel
-O-R′-O-
wobei R′ ein zweiwertiger Rest mit zumindest einem Phenylring ist, und
27d) 5 bis 30 Mol.%, insbesondere 15 bis 25 Mol.% wiederkehrenden Einheiten der Formel

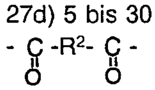

wobei $R^2$ ein zweiwertiger Rest mit zumindest einem Phenylring ist.

Bevorzugte Ausgangsverbindung 27c) ist Hydrochinon und 27d) Terephthalsäure.

Es versteht sich, daß die wiederkehrenden Einheiten auch Alkyl- oder Alkoxyreste mit 1 bis 4 Kohlenstoffatomen, Halogenatome, Phenylreste oder Kombinationen derselben haben können.

28. Thermotrop mesomorphe Polyester, die aufgebaut sind aus
28a) 10 bis 90 Mol.%, insbesondere 10 bis 40 Mol.% wiederkehrenden Einheiten der Formel

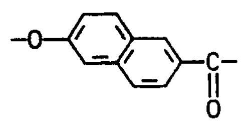

(eine geeignete Ausgangsverbindung ist z.B. 2-Hydroxy-6-naphthalin-carbonsäure),
28b) 5 bis 45 Mol.%, insbesondere 10 bis 40 Mol.% wiederkehrenden Einheiten der Formel
-O-R′-O-
wobei R′ ein zweiwertiger Rest mit zumindest einem Phenylring ist, (eine bevorzugte Ausgangsverbindung ist Hydrochinon), und
28c) 5 bis 45 Mol.%, insbesondere 10 bis 40 Mol.% wiederkehrenden Einheiten der Formel

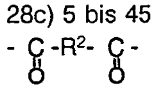

wobei $R^2$ ein zweiwertiger Rest mit zumindest einem Phenylring ist, (eine bevorzugte Ausgangsverbindung ist Terephthalsäure).

Andere bevorzugte Polyester enthalten 60 bis 80 Mol.% der Einheiten 28a), 10 bis 20 Mol.% 28b) und 10 bis 20 Mol.% der Einheiten 28c). Es versteht sich, daß die wiederkehrenden Einheiten auch Alkyl- oder Alkoxyreste mit 1 bis 4 Kohlenstoffatomen, Halogenatome, Phenylreste oder Kombinationen derselben als Substituenten haben können.

29. Thermotrop mesomorphe Polyesteramide, die aufgebaut sind aus
29a) 10 bis 90 Mol.% wiederkehrenden Einheiten der Formel

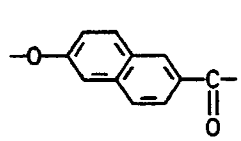

(eine geeignete Ausgangsverbindung ist 2-Hydroxy-6-naphthalin-carbonsäure)
29b) 5 bis 45 Mol.% wiederkehrenden Einheiten der Formel

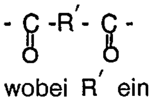

wobei R′ ein zweiwertiger Rest bestehend aus zumindest einem Phenylring oder ein zweiwertiger trans-Cyclohexanring ist, (eine bevorzugte Ausgangsverbindung ist Terephthalsäure),
29c) 5 bis 45 Mol.% wiederkehrenden Einheiten der Formel
$-Y-R^2-Z-$,
wobei $R^2$ ein zweiwertiger Rest bestehend aus zumindest einem Phenylring ist, Y -O-, -NH- oder $-NR^3-$ und Z kann -NH- oder $-NR^3-$sein und $R^3$ eine Alkylgruppe mit 1 bis 6 Kohlenstoffatomen oder ein Phenylring ist, bevorzugte Ausgangsverbindungen 29c) sind 4-Aminophenol oder p-Phenylendiamin), und
29d) 0 bis 40 Mol.% wiederkehrenden Einheiten der Formel
$-O-R^4-O-$
wobei $R^4$ ein zweiwertiger Rest bestehend aus zumindest einem Phenylring ist, (eine bevorzugte Ausgangsverbindung ist Hydrochinon).

Es versteht sich, daß die wiederkehrenden Einheiten auch Alkyl- oder Alkoxyreste mit 1 bis 4 Kohlenstoffatomen, Halogenatome, Phenylreste oder Kombinationen derselben als Substituenten haben können.

Weiterhin ist es allgemein möglich, daß die thermotrop mesomorphen Polymeren wiederkehrende Einheiten beinhalten, die die Bildung von Verzweigungsstellen ermöglichen. Geeignete Ausgangsverbindungen sind z.B. 1,3,5-Trihydroxybenzol, 3,5-Dihydroxybenzoesäure oder 5-Hydroxyisophthalsäure. Solche Polymere sind z.B. in der DE-OS 33 46 549 beschrieben.

Außerdem ist es möglich, daß die Polymeren als Blocksysteme aufgebaut sind, wobei die Polymerketten aus einem odere mehreren Blöcken, die zur Ausbildung von mesomorphen Schmelzen befähigt sind, und eines oder mehren Blöcken eines nicht thermotrop mesomorphen Thermoplasten bestehen.

Es ist weiterhin möglich, auch Mischungen verschiedener thermotrop mesomorpher Polymerer oder Blends aus thermotrop mesomorphen Polymeren und anderen thermoplastischen Polymeren oder Blockpolymeren aus einem oder mehreren Blöcken, die zur Ausbildung einer mesomorphen Schmelze befähigt sind und einem oder mehreren Blöcken eines nicht thermotrop mesomorphen Thermoplasten einzusetzen.

Verfahren zur Herstellung der vorstehend beschriebenen thermotrop mesomorphen Polymeren sind an sich bekannt und in der Literatur, insbesondere auch in den einzelnen, bei den Produkten angeführten Patentanmeldungen beschrieben.

Als Komponente B) enthalten die erfindungsgemäßen Implantatmaterialien 5 bis 70, vorzugsweise 10 bis 65 und insbesondere 25 bis 60 Gew.%, bezogen auf das Gesamtgewicht, eines Apatits.

Apatit wird durch die Formel
$Ca_{10}(PO_4)_6(OH,F,Cl)_2$
dargestellt, wobei weiterhin noch 1 bis 10 Gew.% Carbonationen ($CO_3^{2-}$) enthalten sein können.

Bevorzugt wird als Komponente B) Hydroxylapatit
$Ca_{10}(PO_4)_6(OH)_2$
verwendet, wie er kommerziell im Handel erhältlich ist. Der Apatit B) kann eine gewisse Menge Whitlockit ($Ca_3(PO_4)_2$) enthalten, ohne daß nachteilige Auswirkungen auftreten.

Die Herstellung des Apatits kann z.B. nach den von Aoki et al in Ceramics 10 (7), S.57-66 (1975) beschriebenen Verfahren erfolgen.

Falls der Apatit in Form sphärischer Teilchen eingesetzt wird, weisen diese einen Durchmesser $d_{50}$ -(Zahlenmittel) von 0,05 bis 100 µm, vorzugsweise von 0,1 bis 50 µm auf.

Neben den Komponenten A) und B) können die erfindungsgemäßen Implantatmaterialien als Komponente C noch bis zu 60, vorzugsweise 0,5 bis 50 und insbesondere 2 bis 40 Gew.% zu weiteren, von B) verschiedene Füllstoffe enthalten.

Beispielhaft seien hier Kaolin, gebrannter Ton, Talkum, Glimmer, Calciumsilikat, Feldspat, Sillimanit, Bentonit, Glasflocken, Glaspulver (gepulverter Quarz), Glaskugeln, Calciumcarbonat, Bariumcarbonat, Magnesiumcarbonat, Dolomit, Bariumsulfat, Calciumsulfat, Aluminiumoxid, Antimontrioxid, Magnesiumoxid, Titandioxid, Zinkoxid, Quarz, Flintglas und Diatomeenerde genannt.

Es versteht sich, daß bei physiologisch bedenklichen oder toxischen Füllstoffen darauf zu achten ist, daß der Füllstoff nicht mit dem organischen Gewebe in Kontakt kommt; ganz allgemein werden biologisch verträgliche Füllstoffe bevorzugt.

Zur weiteren Erhöhung der Steifigkeit können als Komponente C) auch faserförmige Füllstoffe eingesetzt werden. Hier seien nur Metallfasern, Kohlenstoffasern und Glasfasern der verschiedenen im Handel erhältlichen Arten erwähnt. Des weiteren können auch anorganische Fasern aus Steinwolle, Zirkonoxid, Aluminium-Silizium-Mischoxiden, Kaliumtitanat, Bariumtitanat, Siliciumcarbid, Aluminiumoxid verwendet werden. Alle diese Produkte sind kommerziell im Handel erhältlich. Weitere mögliche faserförmige Füllstoffe

sind der EP-A 206 600 zu entnehmen.

Besonders hingewiesen sei noch auf Glasfasern, die überwiegend aus Calciumphosphat bestehen. Besonders geeignet sind hierbei Glasfasern, die einen Gesamtgehalt an CaO und $P_2O_5$ von nicht weniger als 15 Gew.% aufweisen, wobei das Molverhältnis von Ca/P im Bereich von 0,3:1 bis 4,0:1, vorzugsweise von 0,8:1 bis 2,0:1 liegt.

Auch Glasfasern, die mit derartigen Calciumphosphatverbindungen beschichtet sind, sind geeignet.

Faserförmige Füllstoffe der beiden letztgenannten Typen wie auch Verfahren zu deren Herstellung sind in der DE-A 35 42 535 beschrieben.

Die Herstellung der erfindungsgemäßen Implantatmaterialien kann am einfachsten dadurch erfolgen, daß man den Apatit B) und ggf. die weiteren Füllstoffe C) in eine Schmelze des thermotrop mesomorphen Polymeren A) einmischt. Dies kann am einfachsten auf einem Extruder oder einer entsprechenden für diesen Zweck bekannten Mischvorrichtung erfolgen.

Nach Herstellung der Mischung können dann durch Spritzguß Implantatmaterialien in fast beliebiger Form hergestellt werden, d.h. die Herstellung ist wesentlich einfacher als bei den bisher bekannten Implantaten auf der Basis von Metallen oder keramischen Werkstoffen, deren Formgebung einen erheblichen Aufwand erfordert.

Die erfindungsgemäßen Implantatmaterialien zeichnen sich durch gute Verträglichkeit mit dem intakten Gewebe in unmittelbarer Umgebung des Implantationsorts bei gleichzeitig sehr hoher Steifigkeit und Festigkeit aus. Insbesondere kann die Steifigkeit des Implantats der des Knochens angepaßt werden, so daß keine größeren E-Modul-Sprünge an den Grenzflächen stattfinden und eine angemessene Belastung des Knochens durch die Endoprothese erfolgt. Außerdem zeichnen sich die erfindungsgemäßen Implantatmaterialien durch eine hohe Strahlungsbeständigkeit aus, wodurch sie effektiv durch $\gamma$-Strahlung sterilisiert werden können. Zudem sind sie, wie bereits erwähnt, besonders einfach und kostengünstig herstellbar.

## Ansprüche

1. Implantatmaterial zum Ersatz von hartem Gewebe, enthaltend als wesentliche Komponenten
   A) 30 bis 95 Gew.% eines thermotrop mesomorphen Polymeren
   B) 5 bis 70 Gew.% eines Apatits
   C) 0 bis 60 Gew.% eines von B) verschiedenen faser- oder teilchenförmigen Füllstoffs.

2. Implantatmaterialien nach Anspruch 1, dadurch gekennzeichnet, daß das thermotrop mesomorphe Polymer ein Polykondensat aus der Gruppe der Polyester, Polyesteramide, Polyesterimide, Polyestercarbonate, Polyetherester, Polyetheresteramide, Polyesteramidimide, Polyestercarbamide und Polyetheresterimide ist.

3. Implantatmaterialien nach den Ansprüche 1 und 2, dadurch gekennzeichnet, daß der Apatit Hydroxyapatit ist.